# EUROPEAN PATENT SPECIFICATION

(11) **EP 1 549 308 B1**
(45) Date of publication and mention of the grant of the patent: **09.08.2006**
(21) Application number: 03748315.3
(22) Date of filing: 17.09.2003
(51) Int. Cl.: A61K 31/435, A61K 31/4985, A61K 31/517, A61P 35/00

(54) **Protecting a patient from possible adverse effects of inhibition of the SHH- signaling pathway by suppressing testosterone**
Schutz eines Patienten vor möglichen Nebenwirkungen entstanden durch die Verhinderung der SHH-CASCADE indem Testosteron unterdrückt wird
Protection d'un patient des effects secondaires de l'inhibition de la voie de signalisation SHH par l'élimination de la testostérone

(30) Priority: 17.09.2002 GB 0221539
(43) Date of publication of application: 06.07.2005
(73) Proprietor: MEDICAL RESEARCH COUNCIL, London W1B 1AL (GB)
(72) Inventor: THOMSON, Axel Andreas, MRC Human Repr. Sci. Uni, Old Dalkeith Road, Edinburgh EH15 4SB (GB)
(74) Representative: Miles, John Stephen
(86) International application number: PCT/GB2003/004117
(87) International publication number: WO 2004/026304

(56) References cited:
- BERMAN DAVID M ET AL: "Medulloblastoma growth inhibition by Hedgehog pathway blockade" SCIENCE (WASHINGTON D C), vol. 297, no. 5586, 30 August 2002 (2002-08-30), pages 1559-1561, XP002266841 ISSN: 0036-8075
- PODLASEK CAROL A ET AL: "Prostate development requires Sonic hedgehog expressed by the urogenital sinus epithelium" DEVELOPMENTAL BIOLOGY, vol. 209, no. 1, 1 May 1999 (1999-05-01), pages 28-39, XP002266842 ISSN: 0012-1606 cited in the application
- BERGMAN D M ET AL: "INHIBITION OF PROSTATE MORPHOLOGENESIS BY THE SONIC HEDGEHOG PATHWAY INHIBITOR CYCLOPAMINE" JOURNAL OF UROLOGY, BALTIMORE, MD, US, vol. 163, no. 4, SUPPL, April 2000 (2000-04), page 204 XP008001018 ISSN: 0022-5347

## Description

The present invention relates to the avoidance of potential undesirable side effects when using inhibitors of SHH signalling such as cyclopamine or its derivatives. It also relates to the use of these compounds in combination with a compound which suppress testosterone in the manufacture of a medicament for treating a proliferative disease such as cancer.

The SONIC HEDGEHOG (SHH)-signalling pathway regulates epithelial-mesenchymal interactions during the development of many organs. SHH protein is synthesised in epithelial cells, and in many situations, acts as a paracrine factor through its receptor PATCHED (PTC) that is expressed in adjacent mesenchymal cells (Bitgood and McMahon, 1995; Roelink et al., 1994; Stone et al., 1996). Disruption of SHH-signalling has provided evidence for important and diverse roles in organogenesis. *Shh* knockout mice exhibit various developmental defects, including cyclopia, neural tube defects and absence of distal limb structures (Chiang et al., 1996). Inhibition of SHH-signalling using cyclopamine (Cy) has further demonstrated the role of SHH-signalling in development of the neural tube (Roelink et al., 1994), gastro-intestinal tract (Ramalho-Santos et al., 2000; Sukegawa et al., 2000), pancreas (Kim and Melton, 1998), and in hair follicle morphogenesis (Chiang et al., 1999). SHH-signalling is also required for branching morphogenesis of the lung (Bellusci et al., 1997a; Pepicelli et al., 1998). *Shh* transcript expression has been reported in the urogenital sinus (UGS) epithelium (Bitgood and McMahon, 1995) where it is required for the formation of external genitalia (Haraguchi et al., 2001; Perriton et al., 2002), and might play an important role in prostate development (Podlasek et al., 1999).

Prostate organogenesis is an androgen-dependent process that involves reciprocal signalling between the epithelium and mesenchyme, but little is known about the molecular mediators that control these epithelial-mesenchymal interactions. *Shh* is expressed in the urogenital sinus (UGS) epithelium, and its importance in prostate growth was demonstrated by antibody blockade of SHH, which abrogated growth of the prostate from male UGS tissue transplanted into male host mice (Podlasek et al., 1999). Podlasek et al also reported that *Shh* transcript expression was upregulated in males in response to androgens, concluding that androgen-induced expression of *Shh* in the UGS is necessary for prostatic induction. However, BMP4, a putative downstream signalling effector of the SHH-signalling pathway (Bitgood and McMahon, 1995), is expressed in the UGS mesenchyme independent of androgen action (Lamm et al., 2001). This discrepancy raised the possibility that androgens might not regulate *Shh* in the growing prostate, prompting us to re-examine this in detail. We have addressed the role of SHH-signalling in prostate organogenesis, by detailed examination of *Shh* and *Ptc* transcript expression, and by disrupting the SHH-signalling pathway in ventral prostate (VPs) grown in vitro. Our results suggest *Shh* and *Ptc* transcript expression is not dependent on androgens, and that SHH-signalling is required for VP growth. Furthermore, we have demonstrated that inhibition of SHH-signalling during VP growth effects epithelial growth, patterning and differentiation, and results in prostatic epithelial ducts reminiscent of human cribiform prostatic intraepithelial neoplasia (PIN).

Thus, the work described suggests for the first time that in the use of compounds that inhibit the SHH signalling pathway for medical treatment of patients it is important for androgens or their effects to be removed.

WO 99/52534 describes the use of steroidal alkaloid derivatives as inhibitors of hedgehog signalling pathways. US Patent No 6,291,516 B1 and WO 01/27135 describes regulators of the hedgehog pathway. WO 02/30462 describes *hedgehog* antagonists.

A first aspect of the invention relates to the use of a compound which suppresses testosterone or its effect in the manufacture of a medicament for protecting a patient from possible adverse effect of a treatment involving inhibition of the SHH-signalling pathway in the patient,

Thus, the invention includes the use of suppression of testosterone or its effect in a patient in order to protect the patient against possible adverse effects on the prostate of the patient of inhibition of the SHH-signalling pathway in the patient.

Inhibition of SHH-signalling, for example using the compounds described in more detail below, is suggested for use in the treatment of various diseases and conditions. For example, WO 02/30462 (incorporated herein by reference), indicates that various such compounds (as discussed in more detail below) are useful for inhibiting angiogenesis and for treating or preventing unwanted cell proliferation, inhibition of SHH-signalling is described for the treatment of diseases or conditions associated with angiogenesis including ocular neovascular disease, age-related macular degeneration, diabetic retinopathy, retinopathy of prematurity, corneal graft rejection, neovascular glaucoma, retrolental fibroplasia, epidemnic keratoconjunctivitis, Vitamin A deficiency, contact lens overwear, atopic keratitis, superior limbic keratitis, pterygium keratitis sicca, sjogrens, acne rosacea, phylectenulosis, syphilis, Mycobacteria infections, lipid degeneration, chemical bums, bacterial ulcers, fungal ulcers, Herpes simplex infections, Herpes zoster infections, protozoan infections, Kaposi sarcoma, Mooren ulcer, Terrien's marginal degeneration, mariginal keratolysis, rheumatoid arthritis, systemic lupus, polyarteritis, trauma, Wegeners sarcoidosis, Scleritis, Steven's Johnson disease, periphigoid radial keratotomy, corneal graph rejection, rheumatoid arthritis, osteoanthritis chronic inflammation (e.g., ulcerative colitis or Crohn's s disease), hemangioma, Osler-Weber-Rendu disease, and hereditary hemorrhagic telangiectasia.

Angiogenesis is also important in certain cancers and metastases, including solid tumours such as rhabomyosarcoma, retinoblastomars, Ewing's sarcoma, neuroblastoma and osteosarcoma, and benign tumours such as acoustic neuroma, neurofibroma, trachonia and pyogenic granulomes.

Other diseases, such as proliferative diseases of the skin including psoriasis and squamous cell carcinoma, and benign prostate hyperplasia (BPH) are also suggested to be treated by inhibitors of SHH-signalling.

For the avoidance of doubt, all of the diseases for treatment with an inhibitor of SHH signalling as described in WO 02/30462 are incorporated herein by reference. Similarly, all of the diseases for treatment with an inhibitor of SHH signalling described in US 6,291,516 B1, WO 01/27135 or WO 99/52534 are incorporated herein by reference.

It is particularly preferred if suppression of testosterone or its effects are used when the patient is being treated for cancer involving inhibition of the SHH-signalling pathway.

Thus, a second aspect of the invention relates to the use of an inhibitor of the SHH-signalling pathway in the manufacture of a medicament for treating a proliferative disease such as cancer in a patient wherein the patient is administered a compound which suppresses testosterone or its effect in the patient.

A third aspect of the invention relates to the use of a compound which suppresses testosterone or its effects in a patient in the manufacture of a medicament for treating a proliferative disease such as cancer in a patient wherein the patient is administered an inhibitor of the SHH-signalling pathway. A forth aspect of the invention relates to the use of a combination of an inhibitor of the SHH-signalling pathway and a compound which suppresses testosterone or its effects in a patient in the manufacture of a medicament for treating a proliferative disease such as cancer in a patient.

The SHH-signalling pathway includes SHH, PTC, Smoothened, Gli-1, Gli-2 and Gli-3. SHH is the ligand for a receptor complex which is made up of PTC and Smoothened. Smoothened is believed to transduce the signal and is a key element of the SHH signalling pathway. Gli-1, Gli-2 and Gli-3 are transcription factors. Further information on the SHH-signalling pathway may be found, for example, in WO 02/30462.

Typically, inhibitors of the SHH-signalling pathway cause changes in growth and/or differentiation. Typically, an inhibitor of the SHH-signalling pathway inhibits the production of PTC and/or BMP-4 (bone morphogenic protein 4) in response to a SHH signal.

Compounds which inhibit SHH or inhibit Smoothened or modulate PTC or which prevent the function of Glis are preferred.

In relation to the methods of the invention, the SHH-signalling pathway may be inhibited by any suitable inhibitor. WO 99/52534, incorporated herein by reference, notes that during biosynthesis, SHH undergoes an autocleavage reaction, mediated by its carboxyl-terminal domain, that produces a lipid-modified amino-terminal fragment responsible for all known SHH signalling activity. SHH autoprocessing causes the covalent attachment of cholesterol to the C-terminus of the N-terminal SHH fragment. WO 99/52534 describes compounds that inhibit the SHH signalling pathway by, for example, disrupting the cholesterol modification of hedgehog proteins and/or which inhibit the bioactivity of hedgehog proteins. In particular, steroidal alkaloids and analogues thereof may be used to interfere with the paracrine and/or autocrine signals produced by SHH, particularly the cholesterol modified forms of the protein. As described in more detail in WO 99/52534, the *Veratrum-*derived compound jervine disrupts such signal and the concomitant biological response of the cell.

The ability of jervine and other steroidal alkaloids to inhibit SHH signalling may be due to the ability of such molecules to interact with PTC or "smoothened"-mediated signal transduction pathway. For instance, the inhibitors may interact with the sterol-sensing domain or domains of the SHH receptor, PTC, or at least interfere with the ability of SHH to interact with its receptor or other molecules associated with the receptor or other proteins involved in SHH-mediated signal transduction. Additionally or alternatively, the effects of jervine on SHH signalling may be the result of disruptions of cholesterol homeostasis which affect cholesterol-mediated autoprocessing of the SHH protein and/or the activity or stability of protein. Thus, other small molecules, steroidal and non-steroidal, which similarly interfere with cholesterol-dependent aspects of PTC activity will disrupt SHH-mediated signals.

The "hedgehog antagonists", and the exemplary compounds described on pages 28 to 38, of WO 99/52534 are hereby specifically incorporated herein by reference as compounds that inhibit the SHH-signalling pathway. It is preferred if the compound which inhibits the SHH-signalling pathway is any of the compounds represented by Formulae I, Ia, II, IIa, III, IIIa, IV, IVa, V, Va, VI, VIa, VII and VIIa of WO 99/52534, all of which are incorporated herein by reference including the definition and preferences for the substituents.

At least some of the compounds are derivatives or analogues of *Solanum-*type or *Veratrum-type* alkaloids, and the compounds include derivatives of jervine, cyclopamine, cycloposine, mukiamine, veratramine, verticine and zygacine, solanidine and chaconine.

WO 02/30462, incorporated herein by reference, also describes inhibitors of the SHH signalling pathway. These, and the exemplary compounds described on pages 33 to 112 of WO 02/30462, are incorporated herein by reference as compounds that inhibit the SHH-signalling pathway. It is preferred if the compound which inhibits the SHH-signalling pathway is any of the compounds represented by Formulae I, Ia, II, IIa, III, IIIa, IV, IVa, V, Va, VI, VIa, VII, VIIa, VIII, VIIIa, IX, IXa, X, Xa, XI, Xia, XII, XIIa, XIII, IIIIa, XIV, XIVa, XV, XVI, XVII, XVIII, XIX, XX, XXI, XXII, XXIII, XXIV and XXV of WO 02/30462, all of which are incorporated herein by reference including the definition and preferences for the substituents. The antagonist *hedgehog* mutants described on pages 93 *et seq,* and the antibody antagonists described on page 102 *et seq,* and the antisense, ribozymes and triplex oligonucleotides described on page 103 *et seq* are also incorporated herein by reference. Similarly, antibody antagonists, antisense, ribozymes and triplex oligonucleotides which inhibit any part of the SHH-signalling pathway such as those directed to PTC, Smoothened, or Gli-1, Gli-2 or Gli-3 may also be useful. The nucleotide and amino acid sequences are available from GenBank as follows: Accession Nos. U43148 (human PTC); L38518 (human SHH); US4401 (human Smoothened); AF316573 (human Gli-1); AB007298 (human Gli-2); and M57609 (human Gli-3), all incorporated herein by reference. The sequences may be used in the design and manufacture of antibodies, antisense nucleic acid, ribozymes and triplex oligonucleotides as is well known in the art.

Preferred hedgehog antagonists include AY9944, triparanol, jervine, cylcopamine and tomatidine (Figure 9), compound A (Figure 10) and compound B (Figure 11).

WO 01/27135, incorporated herein by reference, also describes inhibitors of the SHH-signalling pathway.

The exemplary compounds described on pages 32 to 49, including those of Formulae. I, Ia, II, IIa, III, IIIa, IV, IVa, V, Va, VI, VIa, VII and VIIa, are specifically incorporated herein by reference. Similarly, the compounds described on pages 98 to 133, to the extent that they inhibit the SHH-signalling pathway, are incorporated herein by reference and in particular the cyclopamine and jervine derivatives.

US Patent No 6,291,516 B1, incorporated herein by reference, also describes inhibitors of the SHH-signalling pathway. The compounds described in columns 27 to 35, including those of Formulae I, Ia, II, IIa, III, IIIa, IV, IVa, V, Va, VI, VIa, VII and VIIa are specifically incorporated herein by reference.

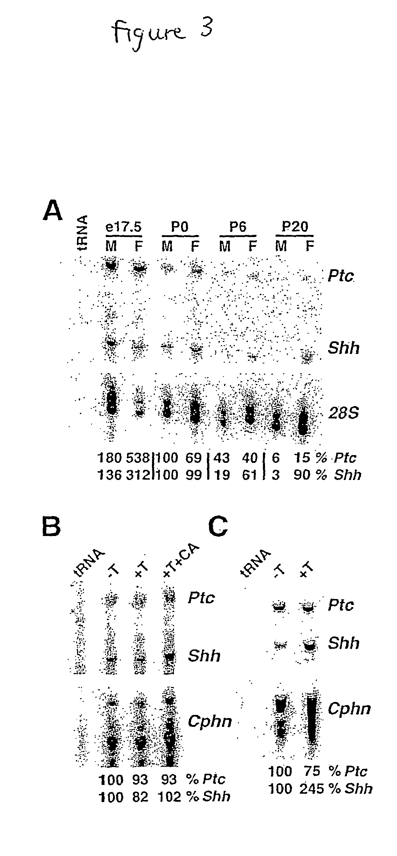

The compounds named SANT-1, SANT-2, SANT-3 and SANT-4 as described in Figure 3A of Chen *et al* (2002) *Proc. Natl. Acad. Sci. USA, 99,* 14071-14076 are Smoothened (Smo) antagonists and so are inhibitors of the SHH-signalling pathway. These structures are incorporated herein by reference.

Typically the SHH signalling pathway is inhibited by the administration of cyclopamine or a derivative thereof to the patient.

The SHH-signalling pathway may be inhibited by agents which interfere with the transcription of the pathway.

Thus, the agent may be an antisense sequence. Antisense sequences which are agents for use in the invention are capable of hybridising to nucleotide sequence. The term "antisense sequence" includes antisense oligonucleotides, such as those between 10 and 30 nucleotides in length, as well as longer sequences. Antisense sequences can be designed by the person skilled in the art by reference to the nucleotide sequences encoding. SHH, PTC, Smoothened, Gli-1, Gli-2, Gli-3.

The term "hybridization" as used herein shall include "the process by which a strand of nucleic acid joins with a complementary strand through base pairing" as well as the process of amplification as carried out in polymerase chain reaction (PCR) technologies.

The present invention also encompasses the use of nucleotide sequences that are capable of hybridising to the sequences that are complementary to the sequences presented herein, or any derivative, fragment or derivative thereof.

The term "variant" also encompasses sequences that are complementary to sequences that are capable of hydridising to the nucleotide sequences presented herein.

Preferably, the term "variant" encompasses sequences that are complementary to sequences that are capable of hydridising under stringent conditions (eg 50°C and 0.2xSSC {1xSSC = 0.15 M NaCl, 0.015 M Na₃citrate pH 7.0}) to the nucleotide sequences presented herein.

More preferably, the term "variant" encompasses sequences that are complementary to sequences that are capable of hydridising under high stringent conditions (eg 65°C and 0.1xSSC {1xSSC = 0.15 M NaCl, 0.015 M Na₃citrate pH 7.0}) to the nucleotide sequences presented herein.

The SHH-signalling pathway may be inhibited by an antibody which binds to any one of SHH, PTC, Smoothened, Gli-1, Gli-2 or Gli-3.

The "antibody" as used herein includes but is not limited to, polyclonal, monoclonal, chimeric, single chain, Fab fragments and fragments produced by a Fab expression library. Such fragments include fragments of whole antibodies which retain their binding activity for a target substance, Fv, F(ab') and F(ab')2 fragments, as well as single chain antibodies (scFv), fusion proteins and other synthetic proteins which comprise the antigen-binding site of the antibody. Furthermore, the antibodies and fragments thereof may be humanised antibodies, for example as described in US-A-239400. Neutralizing antibodies, ie, those which inhibit biological activity of the substance polypeptides, are especially preferred for diagnostics and therapeutics.

Antibodies may be produced by standard techniques, such as by immunisation with the substance of the invention or by using a phage display library.

If polyclonal antibodies are desired, a selected mammal (eg, mouse, rabbit, goat, horse, etc) is immunised with an immunogenic polypeptide bearing a epitope(s) obtainable from an identified agent and/or substance of the present invention. Depending on the host species, various adjuvants may be used to increase immunological response. Such adjuvants include, but are not limited to, Freund's, mineral gels such as aluminium hydroxide, and surface active substances such as lysolecithin, pluronic polyols, polyanions, peptides, oil emulsions, keyhole limpet hemocyanin, and dinitrophenol. BCG (Bacilli Calmette-Guerin) and Corynebacterium parvum are potentially useful human adjuvants which may be employed if purified the substance polypeptide is administered to immunologically compromised individuals for the purpose of stimulating systemic defence.

Serum from the immunised animal is collected and treated according to known procedures. If serum containing polyclonal antibodies to an epitope obtainable from an identified agent and/or substance of the present invention contains antibodies to other antigens, the polyclonal antibodies can be purified by immunoaffinity chromatography. Techniques for producing and processing polyclonal antisera are known in the art. In order that such antibodies may be made, the invention also provides polypeptides of the invention or fragments thereof haptenised to another polypeptide for use as immunogens in animals or humans.

Monoclonal antibodies directed against particular epitopes can also be readily produced by one skilled in the art. The general methodology for making monoclonal antibodies by hybridomas is well known. Immortal antibody-producing cell lines can be created by cell fusion, and also by other techniques such as direct transformation of B lymphocytes with oncogenic DNA, or transfection with Epstein-Barr virus. Panels of monoclonal antibodies produced against orbit epitopes can be screened for various properties; ie, for isotype and epitope affinity.

Monoclonal antibodies may be prepared using any technique which provides for the production of antibody molecules by continuous cell lines in culture. These include, but are not limited to, the hybridoma technique originally described by Koehler and Milstein (1975 *Nature* **256:** 495-497), the human B-cell hybridoma technique (Kosbor *et al* (1983) *Immunol Today* 4: 72; *Cote et al* (1983) *Proc Natl Acad Sci* **80:** 2026-2030) and the EBV-hybridoma technique (Cole *et al* (1985) Monoclonal Antibodies and Cancer Therapy, Alan R Liss Inc, pp 77-96). In addition, techniques developed for the production of "chimeric antibodies", the splicing of mouse antibody genes to human antibody genes to obtain a molecule with appropriate antigen specificity and biological activity can be used (Morrison *et al* (1984) *Proc Natl Acad Sci* **81:** 6851-6855; Neuberger *et al* (1984) *Nature* **312:** 604-608; Takeda *et al* (1985) *Nature* **314:** 452-454). Alternatively, techniques described for the production of single chain antibodies (US Patent No. 4,946,779) can be adapted to produce the substance specific single chain antibodies.

Antibodies may also be produced by inducing *in vivo* production in the lymphocyte population or by screening recombinant immunoglobulin libraries or panels of highly specific binding reagents as disclosed in Orlandi *et al* (1989, *Proc Nail Acad Sci* **86:** 3833-3837), and Winter G and Milstein C (1991; *Nature* **349:** 293-299).

Antibody fragments which contain specific binding sites for the substance may also be generated. For example, such fragments include, but are not limited to, the F(ab')2 fragments which can be produced by pepsin digestion of the antibody molecule and the Fab fragments which can be generated by reducing the disulfide bridges of the F(ab')2 fragments. Alternatively, Fab expression libraries may be constructed to allow rapid and easy identification of monoclonal Fab fragments with the desired specificity (Huse WD *et al* (1989) *Science* **256**: 1275-1281).

By "suppressing testosterone or its effect in the patient" is included reduction in the endogenous level of testosterone (for example by inhibiting its production), inhibition of the conversion of testosterone to dihydrotestosterone (for example by inhibiting testosterone 5α reductase), and blocking of the androgen receptor (eg by using an androgen antagonist). Thus, the testosterone signalling pathway is blocked.

It may also be possible to block the testosterone signalling pathway, for example using a molecule which suppresses production of androgen receptor (AR) such as an AR specific antisense molecule.

Agents which are able to suppress testosterone or its effect are well known in the art and may be considered to be chemical male castration agents. Testosterone may be suppressed by surgical castration of the male but this is less preferred.

In chemical and surgical castration of males level of androgens is reduced but a low level of androgens, produced by the adrenal glands, may be retained. Typically, castrate levels of testosterone are around 2 nmol/l (or 0.5 ng/ml) compared to healthy males which have a normal range of 3.5 to 10.0 ng/ml.

It is particularly preferred if the level of testosterone is suppressed to castrate levels. Preferably, the level of testosterone, or its effect, is suppressed by administering any one or more of a GnRH (LHRH) antagonist, a GnRH (LHRH) agonist, a testosterone (androgen) antagonist or a 5α-reductase inhibitor to the patient.

GnRH (LHRH) agonists include leuprorelin (Prostap supplied by Wyeth which may typically be administered at a level of 3.75 mg every 4 weeks s.c./i.m. or 11.25 mg every 3 months s.c.; buserelin (Suprefact supplied by Shire) which is typically administered at a level of 0.5 mg s.c. every 8 hours or 0.2 mg six times daily intranasally; goserelin (Zoladex supplied by Astra Zeneca) which is typically administered as a 3.6 mg s.c. implant every 28 days or 10.8 mg implant s.c. every 12 weeks; triptorelin (Decapeptyl sr supplied by Ipsen) which is typically administered at a level of 3 mg every 4 weeks; nafarelin (Synarel supplied by Searle); deslorelin (Somagard supplied by Shire); and histrelin/supprelin (Ortho Pharmaceutical Corp/Shire).

GnRH antagonists include teverelix (also known as antarelix); abarelix (Plenaxis supplied by Praecis Pharmaceuticals Inc); cetrorelix (Cetrotide supplied by ASTA Medica) which is typically administered at a level of 0.25-0.3 mg s.c. daily; ganirelix (Orgalutran supplied by Organon) which is typically administered at a level of 0.25 mg daily s.c.

Testosterone (androgen) antagonists include a cyproterone such as cyproterone acetate, (Androcur supplied by Schering Health) and typically administered at a level of 50-100 mg twice or three times daily p.o.; bicalutamide (Carodex supplied by Astra Zeneca) which is typically administered at a level of 50-150 mg per day p.o.; and flutamide (Drogenil supplied by Schering-Plough) which is typically administered at a level of 250 mg three times daily p.o.

Testosterone 5α reductase inhibitors include finasteride (Proscar supplied by MSD) which is typically administered at a level of 5 mg per day p.o.

For the avoidance of doubt, s.c. is subcutaneously; p.o. is per os; and i.m. is intramuscularly.

It is preferred if the patient is male.

It is particularly preferred if the patient is a post-pubescent male.

When the invention is used in the treatment of cancer, the cancer may be any cancer, but it is preferred if the cancer to be treated is a cancer in which the SHH-signalling pathway plays a role in its growth and differentiation.

Cancers in which the SHH-signalling pathway are found include human lung squamous carcinoma, adenocarcinoma, basal cell carcinoma, medulloblastoma, meningioma, hemangioma, rhabdomyosarcoma, glioblastoma, sarcoma, renal carcinoma, thyroid carcinoma, bone cancer, and tumours of the breast, kidney, bladder, ureter, prostate, adrenal gland, stomach and intestines. Treatment of prostate cancer is contemplated but not preferred.

In relation to all of the treatment methods of the invention, the inhibition of the SHH-signalling pathway may take place before or after or at the same time as suppressing testosterone or its effect in the patient. Thus, the inhibitor of the SHH-signalling pathway may be administered to the patient before, at the same time as, or after the patient has been administered a compound which suppresses testosterone or its effect. It is preferred that testosterone or its effect are suppressed before the patient is administered the inhibitor of the SHH-signalling pathway.

A suitable dose or doses of the inhibitor of the SHH-signalling pathway and the compound which suppresses testosterone or its effect is administered to the patient at appropriate intervals in order to give a therapeutic effect.

Conveniently, therapeutic regimes, including the doses and timing of administration, may be prepared for the patient in order for him or her to be administered an appropriate amount of the therapeutic compounds and at an appropriate time to have the desired therapeutic effect. Suitable therapeutic regimes may be prepared for the patient by a physician. Typically, the doses and timing of administration are the same as when the therapeutic compound is used on its own. Thus, known chemical castration regimes may be employed and a therapeutically effective dose and treatment regime used for the inhibitor of the SHH-signalling pathway depending on the disease or condition being treated.

The aforementioned compounds for use in the invention (ie the inhibitor of the SHH-signalling pathway or compound which suppresses testosterone or its effect) or a formulation thereof may be administered by any conventional method including oral and parenteral (eg subcutaneous or intramuscular) injection. The treatment may consist of a single dose or a plurality of doses over a period of time.

Preferably, the formulation is a unit dosage containing a daily dose or unit, daily sub-dose or an appropriate fraction thereof, of the active ingredient.

The compounds for use in the invention will normally be administered orally or by any parenteral route, in the form of a pharmaceutical formulation comprising the active ingredient, optionally in the form of a non-toxic organic, or inorganic, acid, or base, addition salt, in a pharmaceutically acceptable dosage form. Depending upon the disorder and patient to be treated, as well as the route of administration, the compositions may be administered at varying doses.

In human therapy, the compounds for use in the invention can be administered alone but will generally be administered in admixture with a suitable pharmaceutical excipient diluent or carrier selected with regard to the intended route of administration and standard pharmaceutical practice.

For example, the compounds for use in the invention can be administered orally, buccally or sublingually in the form of tablets, capsules, ovules, elixirs, solutions or suspensions, which may contain flavouring or colouring agents, for immediate-, delayed- or controlled-release applications. The compounds of invention may also be administered *via* intracavernosal injection.

Such tablets may contain excipients such as microcrystalline cellulose, lactose, sodium citrate, calcium carbonate, dibasic calcium phosphate and glycine, disintegrants such as starch (preferably corn, potato or tapioca starch), sodium starch glycollate, croscarmellose sodium and certain complex silicates, and granulation binders such as polyvinylpyrrolidone, hydroxypropylmethylcellulose (HPMC), hydroxy-propylcellulose (HPC), sucrose, gelatin and acacia. Additionally, lubricating agents such as magnesium stearate, stearic acid, glyceryl behenate and talc may be included.

Solid compositions of a similar type may also be employed as fillers in gelatin capsules. Preferred excipients in this regard include lactose, starch, a cellulose, milk sugar or high molecular weight polyethylene glycols. For aqueous suspensions and/or elixirs, the compounds of the invention may be combined with various sweetening or flavouring agents, colouring matter or dyes, with emulsifying and/or suspending agents and with diluents such as water, ethanol, propylene glycol and glycerin, and combinations thereof.

The compounds for use in the invention can also be administered parenterally, for example, intravenously, intra-arterially, intraperitoneally, intrathecally, intraventricularly, intrastemally, intracranially, intramuscularly or subcutaneously, or they may be administered by infusion techniques. They are best used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well-known to those skilled in the art.

Formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilised) condition requiring only the addition of the sterile liquid carrier, for example water for injections, immediately prior to use. Extemporaneous injection solutions and suspensions may be prepared from sterile powders, granules and tablets of the kind previously described.

For oral and parenteral administration to human patients, the daily dosage level of the compounds for use in the invention will usually be from 1 to 5000 mg per adult (i.e. from about 0.015 to 75 mg/kg), administered in single or divided doses.

Thus, for example, the tablets or capsules of the compound for use in the invention may contain from 1 mg to 1000 mg of active compound for administration singly or two or more at a time, as appropriate. The physician in any event will determine the actual dosage which will be most suitable for any individual patient and it will vary with the age, weight and response of the particular patient. The above dosages are exemplary of the average case. There can, of course, be individual instances where higher or lower dosage ranges are merited and such are within the scope of this invention.

The compounds for use in the invention can also be administered intranasally or by inhalation and are conveniently delivered in the form of a dry powder inhaler or an aerosol spray presentation from a pressurised container, pump, spray or nebuliser with the use of a suitable propellant, e.g. dichlorodifluoromethane, trichlorofluoromethane, dichlorotetrafluoroethane, a hydrofluoroalkane such as 1,1,1,2-tetrafluoroethane (HFA 134A3 or 1,1,1,2,3,3,3-heptafluoropropane (HFA 227EA3), carbon dioxide or other suitable gas. In the case of a pressurised aerosol, the dosage unit may be determined by providing a valve to deliver a metered amount. The pressurised container, pump, spray or nebuliser may contain a solution or suspension of the active compound, e.g. using a mixture of ethanol and the propellant as the solvent, which may additionally contain a lubricant, e.g. sorbitan trioleate. Capsules and cartridges (made, for example, from gelatin) for use in an inhaler or insufflator may be formulated to contain a powder mix of a compound of the invention and a suitable powder base such as lactose or starch.

Aerosol or dry powder formulations are preferably arranged so that each metered dose or "puff" contains at least 1 mg of a compound for use in the invention for delivery to the patient. It will be appreciated that the overall daily dose with an aerosol will vary from patient to patient, and may be administered in a single dose or, more usually, in divided doses throughout the day.

Alternatively, the compounds for use in the invention can be administered in the form of a suppository or pessary, or they may be applied topically in the form of a lotion, solution, cream, ointment or dusting powder. The compounds for use in the invention may also be transdermally administered, for example, by the use of a skin patch. They may also be administered by the ocular route, particularly for treating diseases of the eye.

For ophthalmic use, the compounds for use in the invention can be formulated as micronised suspensions in isotonic, pH adjusted, sterile saline, or, preferably, as solutions in isotonic, pH adjusted, sterile saline, optionally in combination with a preservative such as a benzylalkonium chloride. Alternatively, they may be formulated in an ointment such as petrolatum.

For application topically to the skin, the compounds for use in the invention can be formulated as a suitable ointment containing the active compound suspended or dissolved in, for example, a mixture with one or more of the following: mineral oil, liquid petrolatum, white petrolatum, propylene glycol, polyoxyethylene polyoxypropylene compound, emulsifying wax and water. Alternatively, they can be formulated as a suitable lotion or cream, suspended or dissolved in, for example, a mixture of one or more of the following: mineral oil, sorbitan monostearate, a polyethylene glycol, liquid paraffin, polysorbate 60, cetyl esters wax, cetearyl alcohol, 2-octyldodecanol, benzyl alcohol and water.

Formulations suitable for topical administration in the mouth include lozenges comprising the active ingredient in a flavoured basis, usually sucrose and acacia or tragacanth; pastilles comprising the active ingredient in an inert basis such as gelatin and glycerin, or sucrose and acacia; and mouth-washes comprising the active ingredient in a suitable liquid carrier.

Generally, in humans, oral or topical administration of the compounds of the invention is the preferred route, being the most convenient. In circumstances where the recipient suffers from a swallowing disorder or from impairment of drug absorption after oral administration, the drug may be administered parenterally, *e.g.* sublingually or buccally.

A third aspect of the invention provides use of a compound which suppresses testosterone or its effect in the manufacture of a medicament for protecting a male patient from possible adverse effects of a treatment involving inhibition of the SHH-signalling pathway in the patient.

A fourth aspect of the invention provides the use of an inhibitor of the SHH-signalling pathway in the manufacture of a medicament for treating a proliferative disease such as cancer in a patient wherein the patient is administered a compound which suppresses testosterone or its effect in the patient.

A fifth aspect of the invention provides the use of a compound which suppresses testosterone or its effect in a patient in the manufacture of a medicament for treating a proliferative disease such as cancer in a patient wherein the patient is administered an inhibitor of the SHH-signalling pathway. In relation to the third, fourth or fifth aspects of the invention, the order of administration may not be critical. Thus, the patient may already have been administered the inhibitor of the SHH-signalling pathway before administration of the compound which suppresses testosterone or its effect in the patient, or is administered the inhibitor of the SHH-signalling pathway at the same time as the inhibitor of the compound which suppresses testosterone or its effect in the patient or will be administered the inhibitor of the SHH-signalling pathway after administration of the compound which suppresses testosterone or its effect in the patient. However, it is preferred if testosterone or its effect is suppressed in the patient before administration of the inhibitor of the SHH-signalling pathway.

A sixth aspect of the invention provides the use of a combination of an inhibitor of the SHH-signalling pathway and a compound which suppresses testosterone or its effect in a patient in the manufacture of a medicament for treating a proliferative disease such as cancer in a patient.

A seventh aspect of the invention provides a therapeutic system for treating a proliferative disease such as cancer in a patient, the system comprising an inhibitor of the SHH-signalling pathway and a compound which suppresses testosterone or its effect in the patient.

An eighth aspect of the invention provides a composition comprising an inhibitor of the SHH-signalling pathway and a compound which suppresses testosterone or its effect in the patient.

A ninth aspect of the invention provides a composition comprising an inhibitor of the SHH-signalling pathway and a compound which suppresses testosterone or its effect in the patient for use in medicine. Thus the composition is packaged and presented for use in medicine, for example as a medicament. The composition may be used in human or veterinary medicine; preferably, it is used in human medicine.

Typically, the composition further comprises a pharmaceutically acceptable carrier. Thus, a pharmaceutical composition (or formulation as it may be termed) comprising an inhibitor of the SHH-signalling pathway, a compound which suppresses testosterone or its effect in the patient and a pharmaceutically acceptable carrier is useful in the practice of the invention. The carrier(s) must be "acceptable" in the sense of being compatible with the composition of the invention and not deleterious to the recipients thereof. Typically, the carriers will be water or saline which will be sterile and pyrogen free. Thus, a tenth aspect of the invention provides a pharmaceutical composition comprising an inhibitor of the SHH-signalling pathway and a compound which suppresses testosterone or its effect in the patient and a pharmaceutically acceptable carrier. Types of compositions (or formulations) are described in more detail above.

The patient is preferably a human although the patient may be any mammal such as a cat, dog, horse, cow, sheep, horse, pig and so on and so has veterinary applications.

For veterinary use, a compound for use in the invention is administered as a suitably acceptable formulation in accordance with normal veterinary practice and the veterinary surgeon will determine the dosing regimen and route of administration which will be most appropriate for a particular animal.

In relation to the second, third, fourth, fifth, sixth, seventh, eighth, ninth and tenth aspects of the invention, the compounds that are preferred in respect of the first aspect of the invention are also preferred for these aspects.

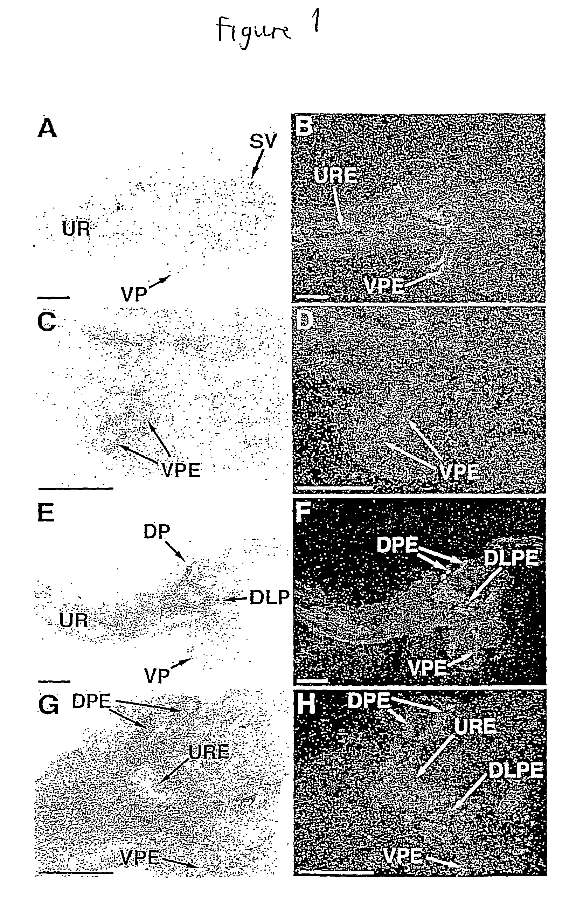

The invention will now be described in more detail by reference to the following Examples and Figures wherein
Figure 1 is an analysis of *Shh* transcript distribution by in situ hybridisation. ³³P-labelled antisense and sense control riboprobes were hybridised to 7 µm paraffin-embedded sections of P0 male rat UGT. (A) Bright field image displaying the UR, VP and seminal vesicles (SV); (B) Dark field image of (A) showing *Shh* transcripts in the urethral epithelium (URE) and bud epithelium (VPE) of the developing VP. (C,D) show higher magnifications of (A,B) respectively. (D) shows *Shh* transcripts expressed in the VPE. (E) Bright field image showing the UR, VP, dorsal prostate (DP) and dorsolateral prostate (DLP); (F) Dark field image of (E) revealing *Shh* transcripts in the URE and bud epithelium (DPE, DLPE and VPE) of the developing DP, DLP and VP lobes. (G,H) show higher magnifications of (E,F) respectively. Scale bar, 500 µm.

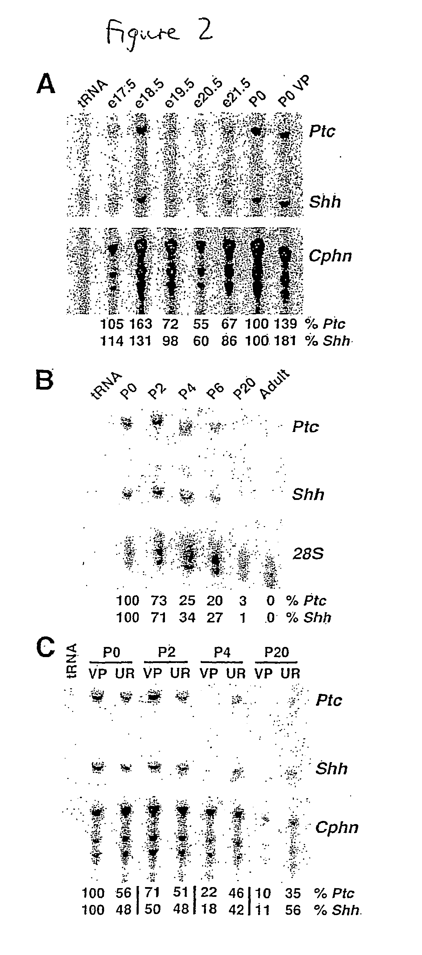

Figure 2 is analysis of *Shh* and *Ptc* transcripts in the male rat UGT, VP and UR by RNase protection assay. RNA was hybridised with ³²P-labelled antisense riboprobes for *Shh, Ptc,* and either *Cyclophilin (Cphn)* or 28S as internal standards. Transcript levels were normalised to *Cphn* or *28S.* Figures below autoradiographs show % transcript abundance, relative to P0 UGT (A) or P0 VP (B,C), calculated as an average of three independent experiments. (A) *Shh* and *Ptc* transcript levels in the developing male UGT. (B) *Shh* and *Ptc* transcript levels in the postnatal VP. (C) Comparison of *Shh* and *Ptc* transcript levels between postnatal VPs and URs.
Figure 3 is a comparison of *Shh* and *Ptc* transcript levels between male and female UGTs, and VPs and URs cultured -/+T. Figures below autoradiographs show % transcript abundance relative to P0 male UGT (A), or -T (B,C), calculated as an average of two experiments. (A) Comparison of *Shh* and *Ptc* transcript abundance between male and female UGTs. (B) Comparison of *Shh* and *Ptc* transcript levels between P0 VPs grown in vitro for six days with either no media supplement (-T), 10⁻⁸ M testosterone (+T), or for five days with 10⁻⁸ M testosterone followed by a one day treatment with 10⁻⁷ M cyproterone acetate (+T +CA). (C) Comparison of *Shh* and *Ptc* transcript levels between P0 female URs cultured for three days with either no media supplement (-T) or with 10⁻⁸ M testosterone (+T).

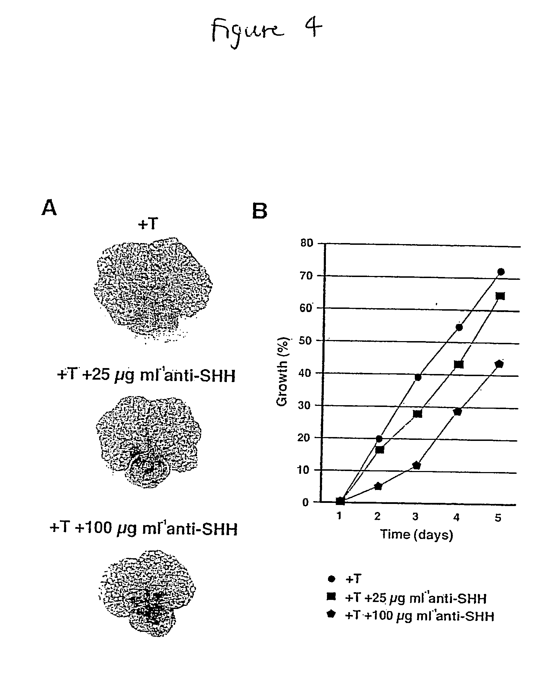

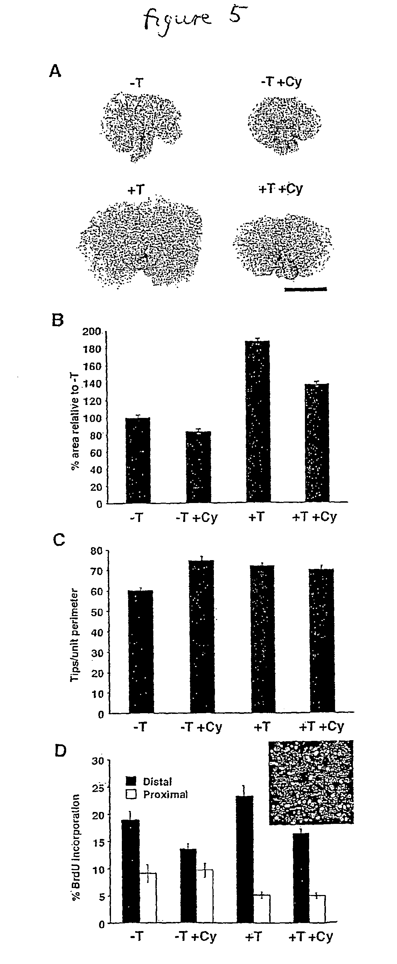

Figure 4 shows the effect of inhibition of SHH-signalling with anti-SHH antibody on VP growth. P0 VPs were grown in serum-free culture for five days in the presence or absence of 10⁻⁸ M T and/or 100 µg ml⁻¹ anti-SHH antibody (5E1). (A) Wholemount images of cultured VPs. Scale bar, 1 mm. (B) Graph showing the mean two-dimensional areas of cultured VPs relative to -T +/- s.e.m.

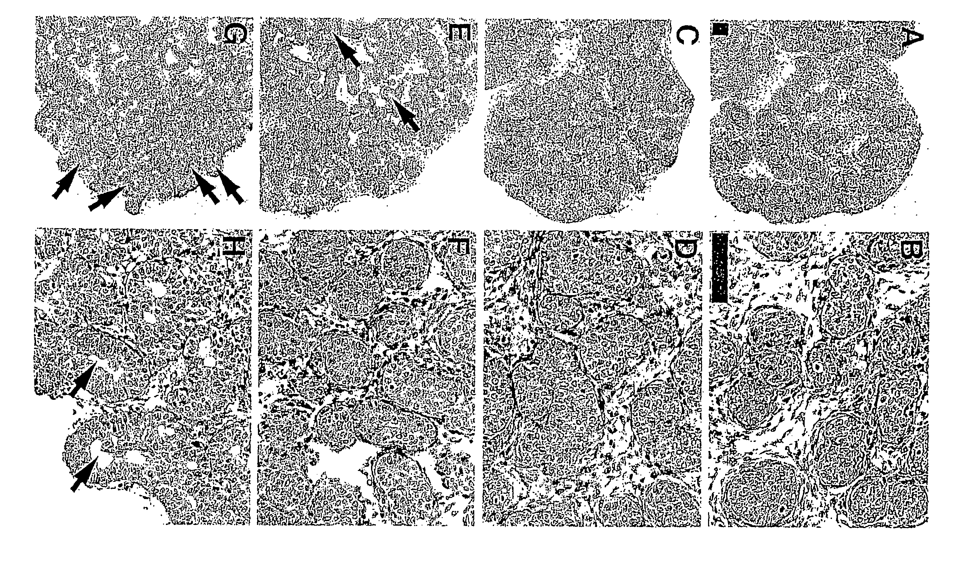

Figure 5 shows the effect of inhibition of SHH-signalling with cyclopamine on VPs grown in vitro. VPs from P0 rats were grown in serum-free culture for six days in the presence or absence of 10⁻⁸ M T and/or 10⁻⁶ M Cy. (A) Whole mount images of VPs on day 6 of culture. Scale bar, 1 mm. (B) Graph showing the mean two-dimensional areas of cultured VPs relative to ―T +/- s.e.m. (C) Graph showing the mean number of epithelial bud tips around the periphery of cultured VPs, expressed as a ratio to organ perimeter (mean buds per 1000 pixels perimeter +/- s.e.m.). (D) Graph showing the mean percentage of proliferating cells in epithelial buds +/s.e.m. (Inset - BrdU incorporation was visualised by immunohistochemistry (green) and localised to the epithelium by immunohistochemistry for pan-cytokeratin (blue); nuclei were counterstained with propidium iodide (red).).

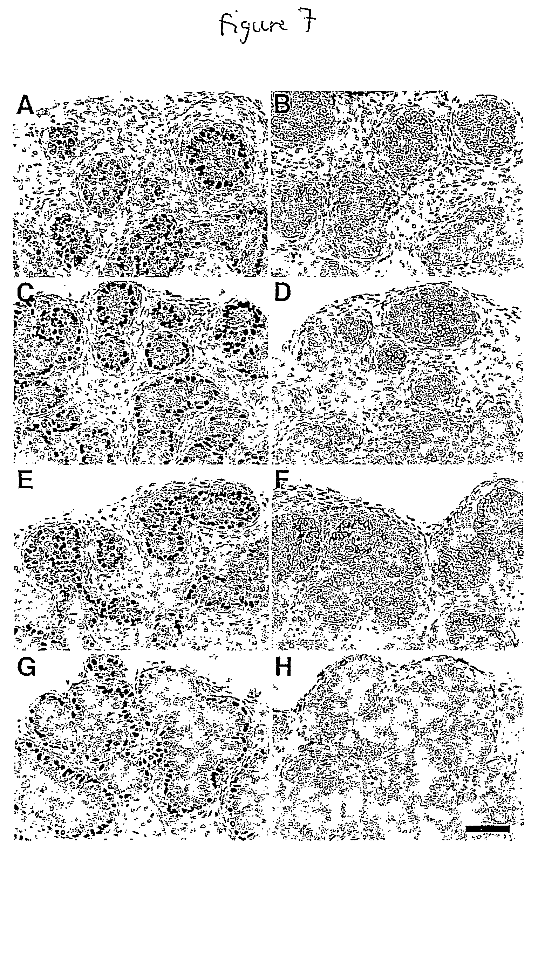

Figure 6 shows the effect of cyclopamine on the histology of prostatic epithelial ducts. Sections of VPs, cultured for six days in the presence or absence of T and/or Cy, were stained with Masson's trichrome. (A,B) organs grown -T, (C,D) organs grown -T +Cy; the addition of Cy in the absence of T did not alter the appearance of epithelial tips. (E,F) organs grown +T; the addition of T resulted in canalisation of areas of ducts proximal to the UR (arrows). (G,H) organs grown +T +Cy; the addition of Cy in the presence of T resulted in canalisation of prostatic ducts throughout the organ, and the appearance of multiple lumens in ducts around the periphery (arrows). Scale bars, 50 µm.

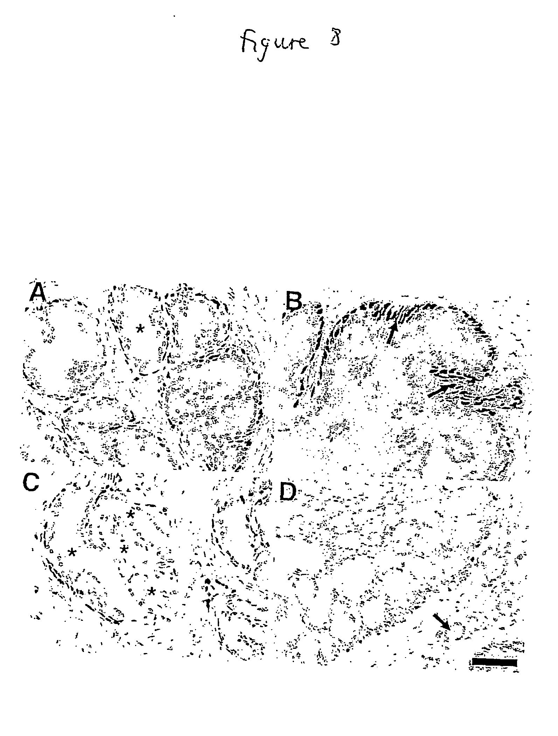

Figure 7 shows the effect of Cy on prostatic epithelial cell differentiation. Paraffin sections of VPs, cultured for six days in the presence or absence of T and Cy, were stained with anti-p63 (A,C,E,G) or anti-cytokeratin 14 (B,D,F,H) antibodies to examine epithelial differentiation. (A,B) VPs grown with no media supplement (-T) exhibited p63 (A) and cytokeratin 14 (CK14) (B) staining throughout distal epithelial tips. p63 (C) and CK14 (D) expression was similar in VPs cultured with -T +Cy (C,D) to VPs cultured - T (A,B). (E,F) VPs cultured +T contained a mixture of distal epithelial tips with staining throughout, and distal tips where p63 (E) and CK14 (F) expression was confined to the basal cell layer. (G,H) In VPs grown +T +Cy, p63 (G) and CK14 (H) expression was confined to the basal cell layer in all tips. CK14 expression (H) was significantly reduced in +T +Cy VPs. Scale bar, 50 µm.

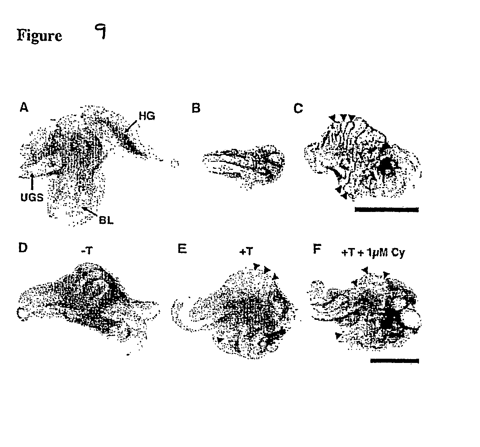

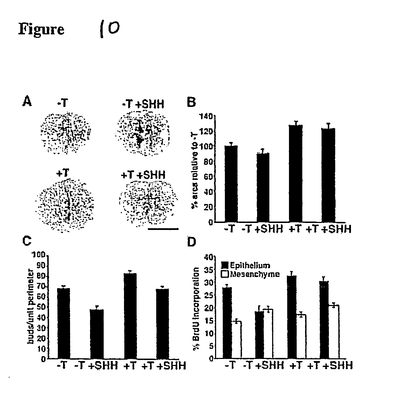

Figure 8 shows p63-immunostaining of human prostatic disease. Paraffin sections of human prostatic needle biopsies were stained with anti-p63 to visualise basal epithelial cells. (A) Normal (asterisk) or slightly hyperplastic ducts, showing only small discontinuities of the basal layer. (B) Expansion of the basal cell layer (arrow) in benign epithelial hyperplasia. (C) Benign cribiform hyperplasia with multiple lumens (asterisks), focal basal cell expansion and localised gaps. (D) High-grade PIN with a much more complex cribiform pattern, expansion of the size of the duct and regular gaps in the basal layer. Adjacent invasive carcinoma (arrow) is p63-negative. Scale bar, 100 µm.

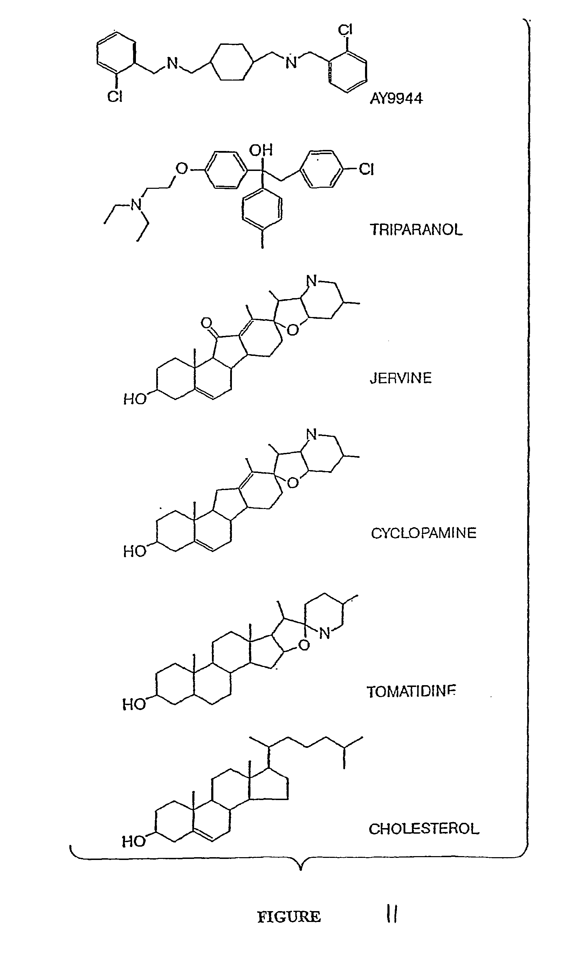

Figure 9 shows prostatic induction in *Shh* null mice and rat UGS grown *in vitro* with cyclopamine. (A) Whole UGT of e17.5 *Shh* null male mouse displaying urogenital sinus (UGS), bladder (BL) and hindgut (HG). e17.5 *Shh* null male mouse UGS before (B) and after (C) growth *in vitro* for 5 days; arrows show prostatic buds. e16.5 male rat UGS grown *in vitro* for 7 days either without media supplement (D), +T (E) or +T+Cy (F); arrows show prostatic buds. Scale bars shown are 1 mm.

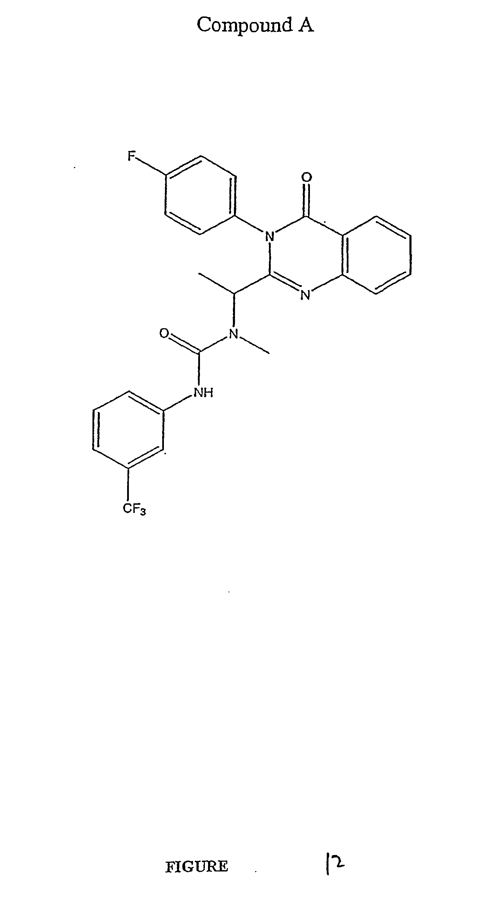

Figure 10 shows the effect of exogenous recombinant SHH protein on VPs grown *in vitro.* VPs from P0 rats were grown *in vitro* for 3 days -/+ T -/+ recombinant SHH. (A) Whole mount images of VPs on day 3 of culture. Scale bar, 1 mm. (B) Graph showing the mean 2D areas of cultured VPs relative to -T +/- s.e.m. (C) Graph showing the mean number of epithelial buds around the periphery of cultured VPs, expressed as a ratio to organ perimeter (mean buds per 1000 pixels perimeter +/- s.e.m.). (D) Graph showing the mean percentage of proliferating cells in distal epithelial buds (at 3 days) and surrounding mesenchyme (at 2 days) +/- s.e.m.

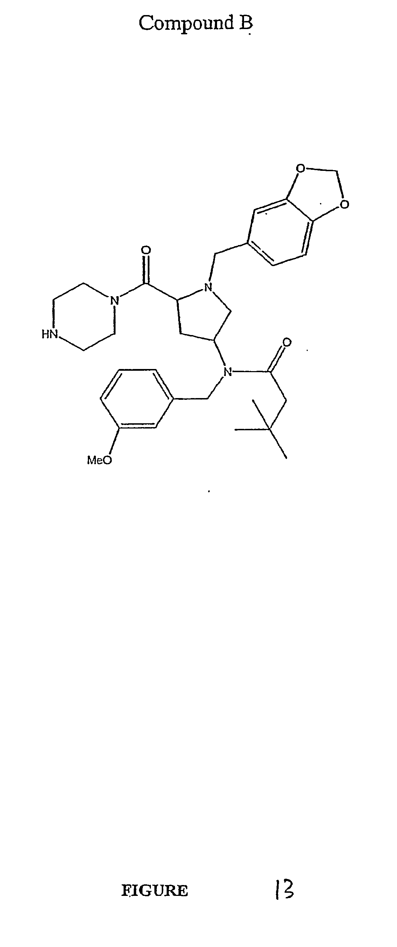

Figure 11 shows the structure of AY9944, triparanol, jervine, cyclopamine, fomatldine and cholesterol.
Figure 12 shows the structure of Compound A from WO 02/30462.
Figure 13 shows the structure of Compound B from WO 02/30462.

### Example 1: Sonic hedgelhog regulates prostatic growth and epithelial differentiation

### Summary

The SONIC HEDGEHOG (SHH)-signalling pathway mediates signalling between epithelium and mesenchyme in several tissues during development and disease. Prostatic organogenesis is dependent on both androgens and epithelial-mesenchymal signalling, and we have investigated the role of SHH-signalling in rat ventral prostate (VP) development. Our data suggests that SHH-signalling is important for VP growth, branching, and mitogenesis and differentiation of prostatic epithelia. We have demonstrated that *Shh* and *Ptc* expression correlates with growth and development of the prostate, and that *Shh* and *Ptc* expression in the VP is not regulated by androgens. SHH-signalling was disrupted with anti-SHH antibody or with cyclopamine, and either treatment inhibited growth of the VP in vitro. Inhibition of SHH-signalling caused an increase in ductal tip number, and a reduction in mitogenesis of ductal tip epithelia. In the presence of testosterone, disruption of SHH-signalling accelerated the canalisation of prostatic epithelial ducts, and resulted in ducts that showed some similarities to cribiform prostatic intraepithial neoplasia (PIN). Epithelial differentiation was examined using p63 and cytokeratin 14 (CK14) as markers, which demonstrated a precocious and aberrant differentiation of the epithelial cells of distal tips. In conclusion, we show that SHH-signalling is required for prostatic growth, and that androgen-stimulated growth in the absence of signalling from the SHH pathway results in aberrant epithelial differentiation reminiscent of PIN.

### Materials and Methods

### RNA Isolation

Whole urogenital tracts (UGTs), VPs and URs were micro-dissected from either outbred Wistar rats or *Shh* null mice or littermates on an outbred CD 1 background (Chiang *et al* (1996) *Nature* **383,** 407-413), where the day of copulatory plug observation was taken as e0.5, and day of birth was designated P0. Total RNA was prepared as described (Chomczynski and Sacchi, 1987).

### RNase Protection Assays

DNA templates for *Shh* and *Ptc* riboprobes were synthesised by RT-PCR from P0 UGT cDNA and subcloned into pBluescript KSII+ (Stratagene, USA). PCR primers were designed using GenBank-published cDNA sequences (*Shh*: GenBank Accession No. NM_017221; Primers: L - ACCGCAGCAAGTATGGCA (SEQ ID No 1), R - TCCAGGAAGGTGAGGAAG (SEQ ID No 2); *Ptc:* GenBank Accession No. AF079162; Primers L - GCATTGGCAGGAGGAGTTGATTGTG (SEQ ID No 3), R - CCACTCGGATGACACTGACA (SEQ ID No 4)). DNA templates for *Cyclophilin (Cphn)* and 28S riboprobes were from Ambion, Inc., USA. ³²P-labelled anti-sense riboprobes were transcribed, and RNase protection assays performed as previously described in detail (Thomson et al., 1997). RNase protected products from *Shh, Ptc, Cphn* and *28S* riboprobes were 269 nucleotides (nt), 322 nt, 103 nt and 155 nt respectively. Gels were imaged using a Storm phosphorimager (Molecular Dynamics, USA) and transcript abundance was determined from the intensities of bands, calculated using ImageQuant V.1.2 (Molecular Dynamics, USA). Transcript abundances were normalised to either *Cphn* or *28S* internal standards (*28S* was used as an internal standard in postnatal samples as *Cphn* expression was found to considerably decrease with age).

### In Situ Hybridisation

In situ hybridisation was performed on 7 µm paraffin-embedded sections using a procedure slightly modified from previously described protocols (Frohman et al., 1990; Thomson and Cunha, 1999). ³³P-labelled anti-sense and control sense riboprobes were synthesised using the same *Shh* DNA template and method as for RNase protection assays. Slides were dipped in G.5 emulsion (Ilford, UK) and developed according to manufacturer's instructions. Sections were examined using bright and dark field microscopy.

### Organ Culture

Serum-free organ culture of P0 Wistar rat VPs was performed as previously described (Sugimura et al., 1996). Culture media was-supplemented with 10⁻⁸ M testosterone and/or either 1 µM, 5 µM or 10 µM cyclopamine, or 100 µg ml⁻¹ anti-SHH antibody (clone 5E1, Developmental Studies Hybridoma Bank, University of Iowa or 0.2 µg/ml recombinant SHH protein (Research Diagnostics, USA)). The two-dimensional area and perimeter of organs was measured from live captured images using NIH Image software.

### Histology and Immunohistochemistry

The histology of sections was examined either by trichrome staining (Masson, 1929), or after immunohistochemical staining. For CK14 and p63 immunostaining, with diamino benzamide (DAB) detection, sections were pressure-cooked in 10 mM citric acid pH 6.0 for two minutes before following a previously published protocol (Thomson et al., 2002). Anti-CK14 antibody was diluted at 1:100, and anti-p63 antibody (Santa Cruz Biotechnologies Inc., USA) was diluted at 1:500. BrdU and pan-cytokeratin co-localisation immunohistochemistry was performed as previously described (Thomson et al., 2002), using 1:300 dilution of anti-BrdU antibody (Fitzgerald Industries International Inc., USA) and 1:200 dilution of anti-pan-cytokeratin antibody (Sigma, UK). BrdU was detected with Alexofluor 488 (Molecular Probes Inc., USA) and pan-cytokeratin was detected with Cy5 (Amersham Biosciences, UK). Mitotic indices were calculated by counting the number of pan-cytokeratin-positive epithelial cells that were also BrdU-positive.

### Results

### Shh mRNA distribution in the male UGT

*Shh* transcripts were localised to the urethral epithelium (URE), and epithelia of the developing ventral prostate (VPE), dorsal prostate (DPE) and dorsolateral prostate (DLPE) of P0 male UGTs, by in situ hybridisation (Fig. 1.). *Shh* transcripts were not observed in the stroma of the UGT. No signal was observed using a control sense riboprobe (data not shown).

The temporal expression patterns of *Shh* and *Ptc* mRNAs were examined by RNase protection assay (RPA)(Fig. 2). *Shh* and *Ptc* transcripts were most abundant at e18.5 in the male UGT (Fig. 2A). Transcript levels were low between e19.5 and e21.5, but were elevated at P0. *Shh* and *Ptc* transcript levels were higher in the VP than the UGT at P0, which may have been due to variation in the proportions of epithelium in these tissues. Analysis of *Shh* and *Ptc* transcripts in the VP was not practical before P0 due to difficulties in microdissection of the VP, as the organ rudiment is small and hard to separate from the UR.

In the postnatal VP, *Shh* and *Ptc* transcript levels decreased with increasing age (Fig. 2B). *Shh* and *Ptc* mRNAs were most abundant at P0 and P2, but by P6 these levels were four-fold lower than at P0. *Shh* and *Ptc* transcripts were almost undetectable at P20, when VP growth rapidly slows, and absent in adults.

A comparison of *Shh* and *Ptc* transcript levels between the VP and UR of postnatal male rats showed that transcripts were more abundant in the VP than UR from P0 to P2, but that by P4 this situation was reversed (Fig. 2C). Unlike in the VP, expression in the UR was maintained beyond P20, separating the developmental pathways of these structures.

### Shh mRNA expression is not androgen-regulated in the VP

As prostate development only occurs in males, and male-specific upregulation of *Shh* mRNA in the mouse UGS had been reported (Podlasek et al., 1999), *Shh* and Ptc transcript levels were compared between male and female rat UGTs (Fig. 3A). RPA analysis showed *Shh* and *Ptc* mRNA expression was approximately two-and-half-fold higher in females than males at e17.5, while at P0 and P6 transcript levels were similar between sexes. At P20, expression of *Shh* and *Ptc* transcripts was minimal in the male but still maintained in the female.

RPAs were performed to analyse *Shh* and Ptc transcripts levels in VPs and URs grown in vitro. VPs were cultured for six days in the presence or absence of T; or for five days with T followed by a one-day treatment with the AR antagonist cyproterone acetate (Fig. 3B). No significant difference in transcript abundance was noted for either *Shh* or *Ptc.* Similarly, in short-term cultures of VPs (seven hours or three days), no changes in transcript levels were observed (data not shown). Analysis of transcript levels in the microdissected URs of female UGTs cultured for three days in the presence or absence of T revealed a two-and-a-half-fold upregulation of *Shh* transcripts (Fig. 3C), although no significant change in Ptc transcript levels was observed.

### Inhibition of SHH-signalling reduces VP growth

P0 VPs were grown in vitro for five days in the presence (+) or absence (-) of T and with (+) or without (-) 100 µg ml⁻¹ anti-SHH antibody (5E1) (Fig. 4A), after which their two-dimensional area was measured (Fig. 4B). The addition of 5E1 to cultures reduced VP growth by 20%, both -/+T (or with a control non-specific IgG, data not shown). This data provides evidence for a direct role for SHH-signalling in VP growth, and demonstrates that signalling through PTC/SMO is likely to be dependent on SHH as a ligand (in the VP).

As obtaining large quantities of 5E1 antibody were not practical, a larger study of SHH-signalling in VP growth was performed using the steroidal alkaloid cyclopamine (Cy), which blocks signalling via PTC/SMO (Incardona et al., 1998). P0 VPs were cultured for six days -/+T, and -/+Cy (Fig. 5A), and their two-dimensional area was then measured (total of 100 VPs from each of four treatment groups and 21 independent experiments) (Fig. 5B). Dose-response experiments showed the optimum concentration of Cy for growth inhibition to be 1 µM. Cultures performed using 0.1 µM Cy showed little or no effect, while 10 µM Cy appeared to be toxic, with organs dying prematurely (data not shown). Disruption of SHH-signalling with Cy significantly inhibited VP growth in the presence and absence of T. The addition of Cy to organs cultured -T resulted in a growth reduction of 16% (-T versus -T +Cy; Student's t-test, P = 3.0e⁻¹⁴), and Cy reduced the growth of VPs cultured +T by 27% (+T versus +T +Cy; Student's t-test, P = 1.4e⁻³⁴) . Therefore, using cyclopamine, inhibition of SHH-signalling had a greater effect on VP growth in the presence of T. Both anti-SHH antibody 5E1 and Cy caused a 50% reduction in T-induced growth of VPs.

### SHH-signalling and epithelial growth

We chose to analyse the effect of Cy on the prostatic epithelia of VPs grown in vitro. Epithelial bud tips situated at the periphery of VPs (total of 60 VPs from each of four treatment groups and 16 independent experiments) were counted and expressed as a ratio to organ perimeter, to account for the changes in organ size after treatments (Fig. 5C). The addition of Cy to organs cultured -T caused a significant increase in the number of peripheral tips per unit perimeter (-T = 60.16 +/- 0.97 mean bud tips per unit perimeter versus -T +Cy = 74.47 +/- 1.07; Student's t-test, P = 1.31e⁻¹⁷). The increase in tip number observed -T +Cy was similar to that observed with +T alone (+T = 71.80 +/- 0.83), however the addition of both T and Cy to cultures did not have an additive effect on peripheral tip number (+T +Cy = 69.84 +/-1.13). It is possible that the number of tips induced by T or Cy were the maximum for the in vitro system, thus additive effects may not be apparent due to the limitations of the system. The addition of Cy to organs cultured +T slightly reduced the number of peripheral tips, when compared to VPs cultured +T alone. However, this reduction showed a low statistical significance (Student's-t test, P = 0.08). An increase in peripheral tip number was also observed in VPs cultured with anti-SHH antibody (5E1) in the absence of T (-T = 62.13 +/- 0.82 versus -T +5E1 = 93.03 +/- 1.08), with a similar peripheral tip number being observed between VPs cultured +T and +T +5E1 (+T = 74.32 +/- 0.69 versus +T +5E1 = 79.21 +/- 1.28).

BrdU incorporation was used to identify proliferating epithelial cells in VPs cultured -/+T and -/+Cy, and to calculate a mitotic index (Fig. 5D). BrdU was added to VPs (four experiments, nine organs in each treatment group) on day six of culture, and its incorporation was visualised by immunohistochemistry. Localisation of BrdU to the epithelium was confirmed by immunohistochemistry for pan-Cytokeratin (Fig. 5D, inset). The percentage of proliferating cells was scored from three images of the peripheral tips of the organs (distal), and three images of the ducts closest to the urethra (proximal), totalling 27 distal and proximal counts for each treatment group. A significant reduction in proliferation was observed in the distal epithelial tips of organs cultured +Cy, both -/+T (-T = 18.92% +/-1.48% mitotic nuclei versus -T +Cy = 13.48% +/- 0.93%, Student's t-test, P = 0.002; +T = 23.24% +/- 1.86% versus +T +Cy = 16.28% +/- 0.74%, Student's t-test, P = 0.0005). No significant difference was observed in the percentage of proliferating cells in the ducts proximal to the UR in response to Cy (data not shown). It was also demonstrated that although T increased proliferation at distal tips, proliferation in proximal ducts was reduced. We conclude that inhibition of SHH-signalling reduces the rate of epithelial cell proliferation at the growing tips of the VP. Therefore, the increased number of distal tips observed in the -T +Cy treatment group was not due to increased epithelial cell proliferation, but more likely due to increased branching.

### Inhibition of SHH-signalling affects canalisation and differentiation of prostatic epithelial ducts

Prostatic ducts grow initially as solid cords, which then canalise, resulting in ducts that contain lumens. Canalisation occurs in a proximal to distal direction (i.e. from the UR outwards), and may be the result of epithelial cell differentiation (Hayward et al., 1996). After growth in vitro, lumens would normally be present in the ducts proximal to the UR (Fig. 6E), but not in the distal tips. Lumens were observed in the proximal ducts of VPs in all treatment groups, but VPs cultured +T +Cy also exhibited lumens in distal tips (Fig. 6G,H). It was also observed that the morphology of ducts was not normal in VPs cultured +T +Cy. Normal prostatic ducts in rats consist of luminal secretory cells with underlying basal cells, forming a single lumen. The architecture of the peripheral ducts of VPs cultured +T +Cy was more complex, with epithelial bridging leading to the appearance of multiple lumens and some expansion of the duct. This cribiform appearance was only focally present in VPs from other treatment groups.

Undifferentiated prostatic epithelial cells at growing tips uniformly express cytokeratin 14 (CK14) (Hayward et al., 1996) and p63 (Parsons et al., 2001; Wang et al., 2000). As epithelial cells differentiate, expression of CK14 (Hayward et al., 1996) and p63 (Parsons et al., 2001; Wang et al., 2000) becomes confined to the basal cell layer. We investigated CK14 and p63 localisation in VPs grown in vitro -/+T and -/+Cy (Fig. 7).

p63 expression was abundant in the epithelial cells of distal tips of organs cultured -T (Fig. 7A), -T +Cy (Fig. 7C) and +T (Fig. 7E). The majority of distal epithelial tips in these organs showed uniform p63 staining, characteristic of the undifferentiated state, while in others there was an increase in p63-positive cells towards the basement membrane. By contrast, in VPs cultured +T +Cy, p63 expression was confined to the basal cell layer of all distal tips (Fig 7G). p63 expression was localised to the basal layer of epithelial cells in the proximal ducts of VPs from all treatment groups.

Expression of CK14 showed similarities to p63, though less basal cells appeared CK14-positive than p63-positive. The majority of distal tips in VPs cultured -T (Fig. 7B) and -T +Cy (Fig. 7D) expressed CK14 broadly, in keeping with undifferentiated epithelial cells. Distal tips with both uniform expression of CK14 and with a defined layer of CK14-expressing basal cells were observed in VPs cultured +T (Fig. 7F). VPs cultured +T +Cy exhibited very few CK14-positive cells in distal tips (Fig. 7H), contrasting with the relative abundance of p63-positive cells. CK14 expression was confined to the basal cell layer in the proximal ducts of VPs from all treatment groups.

Human prostatic needle biopsies with a variety of benign, pre-malignant and malignant lesions were stained with anti-p63 antibody for comparison with our cultured VPs (Fig. 8). Normal human prostatic ducts had a single layer of p63-positive basal cells, and p63-negative luminal cells, forming a single lumen (Fig. 8A). Anti-p63 staining revealed an expansion of the basal cell compartment in benign epithelial hyperplasia (Fig. 8B). Human prostatic ducts showing evidence of high-grade PIN had a discontinuous, single layer of p63-positive basal cells. The luminal cells of the duct formed a complex, cribiform pattern of epithelial bridging that gave the appearance of multiple lumens, and expanded the duct (Fig. 8C). No p63-positive basal cells were present in invasive adenocarcinoma (Fig. 8D). Similarities were noted between the morphology of VPs grown +T +Cy (Fig. 7G) and human high-grade PIN (Fig. SC).

**HH-signalling is not required for prostatic induction.** UGTs were microdissected from e17.5 *Shh* null mice and examined; *Shh* null mice UGTs were smaller than those of their wild-type littermates (not shown), and the hindgut was contiguous with the bladder (Fig 9A). As the prostate was not well developed in either mutant (Fig 9B) or wildtype males at this stage, UGS explants from both *Shh* null mice and their littermates were grown *in vitro* to determine if prostatic budding could be induced (n=3). Prostatic buds were present after 3 days and appeared enlarged after 5 days in both *Shh* null (Fig 9C) and littermate UGS explants (not shown). *Indian hedgehog (Ihh)* is expressed in the VP at much lower levels than *Shh* by RT-PCR (data not shown) and it was possible that an upregulation of *Ihh* (or another *Hh* family member) may have compensated for lack of SHH in *Shh* null UGT explants. Therefore, e16.5 rat UGS explants were grown *in vitro* for 7 days -/+ T and -/+ 1 µM, 5 µM or 10 µM cyclopamine (Cy) (n=4), which blocks signalling via Smoothened and thus all *Hh* family ligands (Chen *et al* (2002) *Genes Dev* **16,** 2743-2748; Incardona *et al* (1998) *Development* **125**, 3553-3562). Prostatic buds were not observed in explants grown -T (Fig 9D); prostatic buds were present in explants grown +T (Fig 9E) and +T +1µM Cy (Fig 9F) at similar levels. Prostatic buds were also present in explants grown +T +5µM Cy and +T +10µM Cy (data not shown), though these concentrations of Cy were toxic and caused premature organ death.

**Effect of exogenous SHH on VP growth and branching.** The effects of adding recombinant SHH to VPs grown *in vitro* was examined (Fig 10). VPs were grown for 3 days -/+T and -/+ recombinant SHH protein (n=6). The addition of SHH to cultures resulted in expansion of the mesenchyme around the periphery of VPs, both -/+ T (Fig 10A). Measurement of the 2D area of the cultured VPs (n=6, 14 VPs from each treatment group)-showed that the addition of SHH caused a small reduction in overall VP growth, both -/+ T, that showed a low statistical significance (-T vs. -T +SHH % area=100 +/- 3.56 vs. 90.12 -/+ 5.26, Student's t-test, P=0.07; +T VS. +T +SHH % area=127.05 +/- 5.12 vs. 123.03 +/- 6.75, Student's t-test, P=0.32) (Fig 10B).

The number of peripheral bud tips from the cultured VPs was counted and expressed as a ratio to perimeter (Fig 10C). The addition of SHH to cultures significantly reduced the number of peripheral bud tips both -/+T, thus having an opposite effect to inhibiting HH-signalling with Cy (-T vs. - T +SHH mean bud tips per 1000 pixels perimeter=68.65 +/- 2.62 vs. 48.09 +/- 3.35, Student's t-test P=3.13e⁻⁵; +T vs. +T +SHH=83.92 +/- 2.25 vs. 68.34 +/- 2.57, Student's t-test P=5.37e⁻⁵).

The proliferative index of the epithelial and mesenchymal cell compartments was calculated for VPs cultured -/+T and -/+ SHH after the addition of BrdU (Fig 10D). Epithelial labelling index was calculated at day 3 of culture while mesenchymal labelling index was calculated on day 2 of culture, since our data indicated that effects on mesenchymal proliferation were more pronounced on day 2 compared to day 3. In VPs grown -T, SHH reduced epithelial cell proliferation by a third (-T vs. -T +SHH % proliferative nuclei=27.95% +/- 1.15% vs. 18.62% +/- 2.06%, Student's t-test P=1.84e⁻⁴), but by only 6% in VPs grown +T, which was not statistically significant (+T vs. ⁺T +SHH % proliferative nuclei= 32.54% +/- 1.65% vs. 30.46% +/- 1.72%, Student's t-test P=0.19). In the mesenchyme, SHH stimuluated proliferation in the presence or absence of T (-T vs -T+SHH % proliferative nuclei= 14.96% +/- 0.89% vs. 19.45% +/-1.12%, Student's t-test P=0.002; +T vs. +T+SHH % proliferative nuclei= 17.51 % +/- 1.22% vs. 20.47% +/- 1.07%, Student's t-test P=0.056).

### Discussion

We have demonstrated here that SHH-signalling regulates growth of the prostate, prostatic epithelia and epithelial differentiation. Inhibition of SHH-signalling retarded growth of VPs cultured in vitro, and reduced mitogenesis, and increased the number of ductal tips in growing prostatic epithelia. In the presence of T, inhibition of SHH-signalling caused aberrant differentiation of prostatic epithelia. We propose that SHH-signalling is an essential regulator of epithelial-mesenchymal interactions in the prostate.

Reciprocal epithelial-mesenchymal interactions play a key role in directing growth of the prostate during development and disease (Cunha and Chung, 1981; Gao et al., 2001; Olumi et al., 1999). Under normal developmental conditions epithelial growth is controlled through androgen-regulated signalling from the mesenchyme (Cunha and Chung, 1981), although the precise mechanism for this is unclear. This paracrine control of epithelial growth is maintained in normal adult prostate, though in prostate cancer it appears that androgenic control of epithelium is autocrine (Gao et al., 2001). Conversely, paracrine signalling from the epithelium patterns stromal differentiation during prostate development, and maintains the mesenchyme/stroma in the normal adult prostate (Cunha et al., 1996; Hayward et al., 1998). It is hypothesised that prostate carcinomas have altered paracrine signalling from the epithelium, as epithelial cells seem unable to induce/maintain differentiation of the mesenchyme/stroma (Cunha et al., 1996).

So far, the signalling pathways identified to regulate epithelial-mesenchymal interactions in prostatic growth are not prostate-specific. The SHH-signalling pathway regulates epithelial-mesenchymal interactions during development in many organs. In the mouse UGT, *Shh* is expressed in the UGS epithelium (Bitgood and McMahon, 1995; Podlasek et al., 1999) and is essential for the formation of external genitalia (Haraguchi et al., 2001; Perriton et al., 2002). It has also been demonstrated that inhibition of SHH-signalling by antibody blockade abrogates branching morphogenesis of the prostate (Podlasek et al., 1999).

The temporal and spatial expression pattern of *Shh* in the male rat UGT suggested a role for *Shh* in growth and development of the VP. *Shh* transcripts were localised to the urethral epithelium and the epithelial buds of the developing prostatic lobes of P0 males, in agreement with earlier studies of *Shh* transcript distribution (Bitgood and McMahon, 1995; Podlasek et al., 1999). RPAs showed *Shh* and *Ptc* transcripts to be most abundant at e18.5, correlating with the visible outgrowth of the urethral epithelium towards the ventral mesenchymal pad (VMP), which defines the onset of VP development. *Shh* and *Ptc* transcript levels were elevated again at P0, associating with the surge of VP growth and branching that occurs in the neonatal period. The temporal expression pattern of *Shh* and *Ptc* in the male UGT was found to reflect that of *Bmp4. Bmp4,* a putative downstream signalling effector of *Shh* (Bitgood and McMahon, 1995), is expressed in the mesenchyme surrounding the growing epithelial buds of the prostate (Lanun et al., 2001). Co- expression of *Shh* and *Bmp4* has been demonstrated at several sites of epithelial-mesenchymal interaction, including the UGS (Bitgood and McMahon, 1995). In the postnatal VP, high levels of *Shh* and Ptc transcripts were observed in the early neonatal period of growth and branching. These levels declined after P6 so that transcripts were almost undetectable by P20, when VP growth slows significantly until quiescence in adulthood (Sugimura et al., 1986). The temporal expression pattern of *Shh* and *Ptc* in the VP correlates with that of *Fgf10,* which is expressed in the mesenchyme surrounding growing epithelial buds (Thomson and Cunha, 1999).

*Shh* and Ptc transcript levels were compared between the VP and UR of postnatal males. While transcript levels decreased in the VP approaching adulthood, expression in the UR was maintained at a similar level throughout development. Thus, the developmental pathways of the VP and UR appear to separate. This data suggests that SHH-signalling is required for growth and branching in the VP, and we propose that maintained SHH-signalling in the UR may be required for continued elongation of the UR into adulthood, or for the formation of urethral glands. Our data also suggests that high levels of *Shh* transcripts reported in the postnatal male mouse UGS (Podlasek et al., 1999) may have originated from the UR.

Comparisons of transcript levels between the male and female UGT demonstrated approximately two-and-a-half-fold more *Shh* and *Ptc* mRNA in the female than male at *e*17.5. Transcript levels were similar at P0 and P6, but at P20 there were significantly more *Shh* and *Ptc* transcripts in the female than the male. The elevated level of transcripts in the P20 female versus the male could reflect anatomical differences in our definition of the UGT. Here, we define the female UGT as UGS (i.e. UR) only, and male UGT as UGS plus accompanying prostatic lobes and SVs. Hence, our female UGT RNA samples contain a higher proportion of urethral RNA than our RNA samples from male UGTs. As the UR maintains *Shh* and *Ptc* mRNA expression to at least P20 (Fig. 2C), the higher levels of *Shh* and *Ptc* transcripts in a female UGT RNA samples could be due to a higher relative proportion of urethral RNA. Our data was found to contradict that of Podlasek et al (1999) who reported seven-fold higher expression of *Shh* in male UGS than female UGS of e19 mice (Podlasek et al., 1999). The inclusion of the developing vagina with the UGT diluting *Shh* transcript abundance in female RNA samples, in the study by Podlasek et al (1999), could explain this discrepancy.

To determine whether SHH-signalling was regulated by androgens in the VP, *Shh* and *Ptc* transcript levels were compared between VPs cultured in vitro for six days -/+T, or for five days +T followed by one day with the AR antagonist cyproterone acetate. No significant difference in transcript levels were observed over this time course, or between organs treated -/+T for seven hours or three days (data not shown). This indicated that *Shh* and *Ptc* mRNA expression is unlikely to be dependent on androgens in the VP. In a previous study we have shown that FGF10 also acts independently of androgen action in VP growth (Thomson and Cunha, 1999). This suggests that while it is known that prostate development is dependent on androgens, the mechanism may be indirect on some signalling pathways. We have recently shown that mesenchymal-epithelial interactions during prostatic induction may be regulated by androgens controlling the differentiation of a smooth muscle layer (Thomson et al., 2002).

Podlasek et al, reported a five-fold increase in *Shh* transcripts in e15 male mouse UGS explants cultured for three days with dihydrotestosterone. This could be specific to the UGS, since androgen receptor (AR) expression does not appear in prostatic bud epithelium until birth (Hayward et al., 1996). In keeping with this, we reported a two-and -a-half-fold upregulation of *Shh* transcripts in response to T in the URs of female UGTs. This was not matched by upregulation of *Ptc,* bringing into question the functional significance of the observation.

It has been observed that *Shh* null mice do not express *Nkx3.1* in the UGS (Schneider et al., 2000). *Mkx3.1* is the earliest known marker of prospective prostatic epithelium, and is expressed exclusively in the UGS of males, in regions where prostatic buds arise from the UGS (Bhatia-Gaur et al., 1999). *Nkx3.1* is not required for prostatic induction, since *Nkx3*. *1* null mutants still have a prostate (Bhatia-Gaur et al., 1999; Schneider et al., 2000), but its expression is required for epithelial differentiation during development, and correct function of the prostate during adulthood (Bhatia-Gaur et al., 1999). As *Nkx3.1* is only expressed in the male UGS, it is likely to be androgen-regulated, and as expression occurs before functional AR appears in the epithelium, this androgen-regulation is likely to be mediated via the mesenchyme (Bhatia-Gaur et al., 1999). Current literature suggests that androgens up-regulate *Shh* expression in males (Podlasek et al., 1999), which in turn induces *Nkx3.1* expression. As we find *Shh* transcripts in the UGS of both males and females, and that there is no male-specific upregulation of *Shh* expression, we propose that *Shh* is a permissive, rather than an inductive, factor for *Nkx3.1* expression in the male UGS.

A direct role for SHH in VP growth and development was demonstrated by inhibition of the SHH-signalling pathway. Blocking SHH-signalling using both anti-SHH antibody and Cy resulted in a reduction in growth of VPs. Growth reduction resulting from antibody blockade confirmed that, during VP growth and development, signalling via PTC/SMO is dependent on SHH as a ligand. *Ihh,* but not *Dhh* transcripts, were observed in the VP, by RT-PCR, but *Ihh* transcript levels were much lower than those of *Shh* (data not shown). It has been demonstrated that SHH is a more potent peptide than IHH (Pathi et al., 2001), and we propose that SHH is the main regulator of signalling via PTC/SMO in the VP. Our results concur with experiments showing that implantation of an anti-SHH bead into the prostatic anlagen of the e15 mouse UGS, grafted under kidney capsule of a male host, abrogated prostate growth and development (Podlasek et al., 1999).

Inhbition of SHH-signalling increased the number of epithelial bud tips around the periphery of VPs, despite a reduction in epithelial cell proliferation, suggesting that the increase was due to augmented branching. In mesenchyme-free lung explants, increased epithelial cell proliferation is not required for branching, but is required for elongation (Nogawa et al., 1998). If the mitogenic effects of SHH on both epithelium and mesenchyme in the lung (Bellusci et al., 1997b) are operative in the prostate, SHH-signalling could act to promote mitogenesis and inhibit branching, which would allow elongation of epithelial bud tips. A significant increase in the number of ductal tips and branch points has also been observed in the VPs of *Bmp4* haploinsufficient mice (Lamm et al., 2001). *Bmp4* is postulated to inhibit lateral branching, thereby allow elongation, of prostatic epithelial ducts (Lamm et al., 2001). *Shh* and *Bmp4* are found co-expressed at several sites of epithelial-mesenchymal interaction during development (Bitgood and McMahon, 1995), and it has been demonstrated that *Shh* can modulate the expression of *Bmp4* (Haraguchi et al., 2001). We suggest that *Shh* might operate upstream of *Bmp4* in a pathway that inhibits branch formation in the prostate.

Epithelial prostatic ducts initially grow out as solid cords, which then undergo canalisation. Canalisation begins at the urethral end of the prostatic duct (proximal) and proceeds towards the distal portion of the duct (Hayward et al., 1996). We observed that canalisation had occurred at the distal tips of VPs cultured +T +Cy, whereas in VPs cultured -T, -T +Cy and +T, canalisation was only evident in proximal ducts, this being most advanced in VPs cultured +T. This suggested that in the presence of T inhibition of SHH-signalling accelerated canalisation, but that disruption of SHH-signalling alone was not sufficient. We propose that SHH-signalling inhibits canalisation of prostatic epithelial ducts in vivo, and so plays an opposing role to T, which promotes canalisation (Hayward et al., 1996).

The morphology of the distal tips of VPs cultured +T +Cy was complex, with a well-developed cribiform pattern and multiple lumens. In human disease, this cribiform pattern can be seen in severe forms of benign epithelial hyperplasias and in high-grade PIN, the precursor of invasive carcinoma. One of the features used in differential diagnosis is the presence of normal nuclei in hyperplasia, contrasting with the enlarged, variable nuclei with visible nucleoli in PIN. However, the latter features are also seen in models of prostate development, and were a consistent feature in all our experiments. Another feature of significant hyperplasia of luminal cells is that it is often accompanied by a corresponding expansion of the basal compartment (Epstein, 1992), which is well demonstrated by p63 immunostaining (Fig. 8B). On the other hand, in lesions of PIN there is generally a single layer of basal cells, often with large gaps (Fig. 8C), as in our VPs cultured +T +Cy (Fig. 7G). Finally, benign epithelial hyperplasia does not generally lead to an expansion of the duct, unlike cribiform PIN (Fig. 8C) and in our +T +Cy cultured VPs (Fig. 7G). These observations suggest that prostatic epithelia of our VPs, grown in vitro +T +Cy, recapitulate some of the features of PIN.

There is a clear temporal and spatial relationship between canalisation of prostatic ducts and epithelial cell differentiation (Hayward et al., 1996). Differentiation of the epithelial cells of immature prostatic ducts into the luminal and basal cells found in mature ducts is marked by changes in CK and p63 expression (Hayward et al., 1996; Wang et al., 2000). Undifferentiated prostatic epithelial cells uniformly express CK5, CK8, CK14, CK18 and p63. Basal cells maintain expression of CK5, CK14, and p63, and lose expression of CK8 and CK18, while luminal cells maintain CK8 and CK18 expression, and lose CK5, CK14 and p63 expression (Hayward et al., 1996; Wang et al., 2000). Immunohistochemistry for p63 revealed that the epithelial cells in all distal tips of VPs cultured +T +Cy had undergone differentiation. A mixture of distal tips, containing either undifferentiated epithelial cells or differentiated basal cells, were present in VPs from all other treatment groups. This suggested that inhibition of SHH-signalling in the presence of T accelerated epithelial cell differentiation. The expression pattern of CK14 was similar to that of p63 except that the majority of cells in the distal tips of VPs cultured +T +Cy failed to express CK14, although those that did were more basal, indicating differentiation. The absence of a complete layer of CK14-positive basal cells is well recognised in human prostate, where variations in expression of keratin subtypes is well described (Abrahams et al., 2002; Freeman et al., 2002). The functional significance of these variations in keratin profile is unknown. Nevertheless, fewer CK-positive cells are generally seen in PIN than in normal glands ((Bostwick and Brawer, 1987), personal observation, P. H.), and the relative paucity of CK14-positive cells in VPs cultured +T +Cy is again reminiscent of PIN. Complete loss of basal cells has long been established as an intrinsic feature of prostatic adenocarcinoma (Totten et al., 1953). This is one of the key features exploited by diagnostic pathologists in the differential between atypical, but benign, lesions and prostatic adenocarcinoma, as the demonstration of basal cells excludes invasive disease. The basal layer is often attenuated and difficult to identify by standard morphology, hence the value of immunocytochemical markers (Wojno and Epstein, 1995). Our data suggest that p63 may be more consistently expressed than keratins, and this could be of value in diagnostic pathology. Our results have shown that SHH-signalling is likely to be independent to androgen action in VP growth. However, co-involvement of SHH-signalling and androgens during prostatic epithelial differentiation is inferred; inhibition of SHH-signalling effects differentiation of distal epithelial tips differently in the presence to the absence of T. This might be related to the higher growth rate of VPs in the presence versus the absence of T. We have shown that the epithelial cells of distal tips proliferate faster in the presence of T (Fig. 5D), so perhaps the effect of inhibiting SHH-signalling only shows an effect in more rapidly growing cells. No obvious effect on differentiation was observed in the proximal ducts of VPs, whose cells are dividing much more slowly than the cells of distal tips after six days in culture. We propose that any interaction between SHH-signalling and androgens during prostatic epithelial differentiation is likely to be indirect and complex, and based on current evidence we are not able to propose a mechanism by which this could occur.

Inappropriate activation of the SHH-signalling pathway has been implicated in several types of tumour (reviewed in (Ruiz i Altaba et al., 2002)). Our data is novel in that inactivation of the SHH-signalling pathway during growth resulted in lesions suggestive of disease. The SHH-signalling pathway may control other genes implicated in prostatic disease. We have already discussed that *Shh* may be a permissive factor for *Nkx3.1* expression during prostatic epithelial growth. In addition to developmental defects, *Nkx3.1* mutant mice develop lesions reminiscent of PIN (Bhatia-Gaur et al., 1999; Kim et al., 2002), as in our +T +Cy cultured VPs.

By disrupting SHH-signalling, we have demonstrated that SHH-signalling is required for prostatic growth, and may inhibit branching morphogenesis. Inhibition of SHH-signalling during growth led to precocious and aberrant differentiation of epithelial cells, resulting in lesions akin to human cribiform PIN. This paper provides new information on the developmental effects of disrupting SHH-signalling during growth, and lends evidence to the theory that inappropriate regulation of signalling pathways active during normal development can be implicated in disease processes.

### Example 2: Treatment of basal cell carcinoma

A male patient presenting with basal cell carcinoma is administered leuprorelin (3.75 mg every four weeks intramuscularly) until castrate levels of testosterone are reached (0.5 ng/ml). The patient is then administered cyclopamine in a dosage regime to ameliorate the basal cell carcinoma.

### Example 3: Treatment of glioblastoma

A male patient presenting with glioblastoma is administered flutamide (250 mg three times daily per os) to suppress the effects of testosterone. The patient is then administered an inhibitor of the SHH-signalling pathway in a dosage regime to ameliorate the glioblastoma.

### REFERENCES

**Abrahams, N. A., Ormsby, A. H. and Brainard, J.** (2002). Validation of cytokeratin 5/6 as an effective substitute for keratin 903 in the differentiation of benign from malignant glands in prostate needle biopsies. *Histopathology* **41**, 35-41.
**Bellusci, S., Furuta, Y., Rush, M. G., Henderson, R., Winnier, G. and Hogan, B. L. (1997a).** Involvement of Sonic hedgehog (Shh) in mouse embryonic lung growth and morphogenesis. *Development* **124,** 53-63.
**Bellusci, S., Grindley, J., Emoto, H., Itoh, N. and Hogan, B. L.** (1997b), Fibroblast growth factor 10 (FGF10) and branching morphogenesis in the embryonic mouse lung. *Development* **124,** 4867-78.
**Bhatia-Gaur, R., Donjacour, A. A., Sciavolino, P. J., Kim, M., Desai, N., Young, P., Norton, C. R., Gridley, T., Cardiff, R. D., Cunha, G. R. et al.** (1999). Roles for Nkx3.1 in prostate development and cancer. *Genes Dev* 13, 966-77.
**Bitgood, M. J. and McMahon, A. P.** (1995). Hedgehog and Bmp genes are coexpressed at many diverse sites of cell-cell interaction in the mouse embryo. *Dev Biol* **172**, 126-38.
**Bostwick, D. G. and Brawer, M. K.** (1987). Prostatic intra-epithelial neoplasia and early invasion in prostate cancer. *Cancer-* **59**, 788-94.
**Chiang, C., Litingtung, Y., Lee, E., Young, K. E., Corden, J. L.**, **Westphal, H. and Beachy, P. A.** (1996). Cyclopia and defective axial patterning in mice lacking Sonic hedgehog gene function. *Nature* **383**, 407-13.
**Chiang, C., Swan, R. Z., Grachtchoulc, M., Bolinger, M., Litingtung, Y., Robertson, E. K., Cooper, M. K., Gaffield, W., Westphal, H., Beachy, P. A. et al.** (1999). Essential role for Sonic hedgehog during hair follicle morphogenesis. *Dev Biol* **205,** 1-9.
**Chomezynski, P. and Sacchi, N.** (1987). Single-step method of RNA isolation by acid guanidinium thiocyanate-phenol-chloroform extraction. *Anal Biochem* **162**, 156-9.
**Cunha, G. R. and Chung, L. W.** (1981). Stromal-epithelial interactions--I. Induction of prostatic phenotype in urothelium of testicular feminized (Tfm/y) mice. *J Steroid Biochem* **14**, 1317-24.
**Cunha, G. R., Hayward, S. W., Dahiya, R. and Foster, B. A.** (1996). Smooth muscle-epithelial interactions in normal and neoplastic prostatic development. *Acta Anat* **155**, 63-72.
**Epstein, J. I.** (1992). Differential diagnosis in pathology:urologic disorders. New York: Igaku-Shoin.
**Freeman, A., Treurnicht, K., Munson, P., Miller, K. and Parkinson, M.** C. (2002). A comparison of basal cell markers used in the prostate. *Histopathology* **40**, 492-4.
**Frohman, M. A., Boyle, M. and Martin, G. R.** (1990). Isolation of the mouse Hox-2.9 gene; analysis of embryonic expression suggests that positional information along the anterior-posterior axis is specified by mesoderm. *Development* **110***,* 589-607.
**Gao, J., Arnold, J. T. and Isaacs, J. T.** (2001). Conversion from a paracrine to an autocrine mechanism of androgen-stimulated growth during malignant transformation of prostatic epithelial cells. *Cancer Res* **61**, 503 8-44.
**Haraguchi, R., Mo, R., Hui, C., Motoyama, J., Makino, S., Shiroishi, T., Gaffield, W**. **and Yamada, G**. (2001). Unique functions of Sonic hedgehog signaling during external genitalia development. *Development* **128,** 4241-50.
**Hayward, S. W., Baskin, L. S., Haughney, P. C., Cunha, A. R., Foster, B. A., Dahiya, R., Prins, G. S. and Cunha, G. R.** (1996). Epithelial development in the rat ventral prostate, anterior prostate and seminal vesicle. *Acta Anat* **155,** 81-93.
**Hayward, S. W., Haughney, P. C., Rosen, M. A., Greulich, K. M., Weier, H. U., Dahiya, R. and Cunha, G. R.** (1998). Interactions between adult human prostatic epithelium and rat urogenital sinus mesenchyme in a tissue recombination model. *Differentiation* **63**, 131-40.
**Incardona, J. P., Gaffield, W., Kapur, R. P. and Roelink, H.** (1998). The teratogenic Veratrum alkaloid cyclopamine inhibits sonic hedgehog signal transduction. *Development* **125,** 3553-62.
**Kim, M., Bhatia-Gaur, R., Banach-Petrosky, W., Desai, N., Wang, Y., Hayward, S. W., Cunha, G. R., Cardiff, R. D., Shen, M. M. and Abate-Shen, C.** (2002). Nkx3.1 Mutant Mice Recapitulate Early Stages of Prostate Carcinogenesis. *Cancer Res* **62**, 2999-3004.
**Kim, S. K. and Melton, D. A.** (1998). Pancreas development is promoted by cyclopamine, a hedgehog signaling inhibitor. *Proc Natl Acad Sci U S A* **95**, 13036-41. -
**Lamm, M. L., Podlasek, C. A., Barnett, D. H., Lee, J., Clemens, J. Q., Hebner, C. M. and Bushman, W.** (2001). Mesenchymal factor bone morphogenetic protein 4 restricts ductal budding and branching morphogenesis in the developing prostate. *Dev Biol* **232,** 301-14.
**Masson, P.** (1929). Some histological methods. Trichrome staining and their preliminary techniques. *Bull Int Assoc Med.* **12,** 75.
**Nogawa, H., Morita, K. and Cardoso, W.** (1998). Bud Formation Precedes the Appearance of Differential Cell Proliferation During Branching Morphogenesis of Mouse Lung Epithelium In Vitro. *Dev Dy*n **213,** 228-35.
**Olumi, A. F., Grossfeld, G. D., Hayward, S. W., Carroll, P. R., Tlsty, T. D. and Cunha, G. R.** (1999). Carcinoma-associated fibroblasts direct tumor progression of initiated human prostatic epithelium. *Cancer Res **59**,* 5002-11.
**Parsons, J. K., Gage, W. R., Nelson, W. G. and De Marzo, A. M. (2001).** p63 protein expression is rare in prostate adenocarcinoma: implications for cancer diagnosis and carcinogenesis. *Urology* **58**, 619-24.
**Pathi, S., Pagan-Westphal, S., Baker, D. P., Garber, E. A., Rayhorn, P., Bumcrot, D. A., Tabin, C. J., Blake Pepinsky, R. and Williams, K. P.** (2001). Comparative biological responses to human Sonic, Indian and Desert hedgehog. *Mech Dev* **106,** 107-17.
**Pepicelli,** C. **V., Lewis, P. M. and McMahon, A. P.** (1998). Sonic hedgehog regulates branching morphogenesis in the mammalian lung. *Curr Biol* **8**, 1083-6.
**Perriton, C. L., Powles, N., Chiang, C., Maconochie, M. K. and Cohn, M. J.** (2002). Sonic hedgehog Signaling from the Urethral Epithelium Controls External Genital Development. *Dev Biol* **247**, 26-46.
**Podlasek, C. A., Barnett, D. H., Clemens, J. Q., Bak, P. M. and Bushman, W.** (1999). Prostate development requires Sonic hedgehog expressed by the urogenital sinus epithelium. *Dev Biol* **209**, 28-39.
**Ramalho-Santos, M., Melton, D. A. and McMahon, A. P.** (2000). Hedgehog signals regulate multiple aspects of gastrointestinal development. *Development* 127*,* 2763-72.
**Roelink, H., Augsburger, A., Heemskerk, J., Korzh, V., Norlin, S., Ruiz i Altaba, A., Tanabe, Y., Placzek, M., Edlund, T., Jessell, T. M. et al.** (1994). Floor plate and motor neuron induction by vhh-1, a vertebrate homolog of hedgehog expressed by the notochord. *Cell* **76**, 761-75.
**Ruiz i Altaba, A., Sanchez, P. and Dahmane, N.** (2002). Gli and Hedgehog in Cancer: Tumours, Embryos and Stem Cells. *Nat Rev Cancer* **2,** 361-72.
**Schneider, A., Brand, T., Zweigerdt, R. and Arnold, H.** (2000). Targeted disruption of the Nkx3.1 gene in mice results in morphogenetic defects of minor salivary glands: parallels to glandular duct morphogenesis in prostate. *Mech Dev* **95**, 163-74.
**Stone, D. M., Hynes, M., Armanini, M., Swanson, T. A., Gu, Q., Johnson, R. L., Scott, M. P., Pennica, D., Goddard, A., Phillips, H. et al.** (1996). The tumour-suppressor gene patched encodes a candidate receptor for Sonic hedgehog. *Nature* **384,** 129-34.
**Sugimura, Y., Cunha, G. R. and Donjacour, A. A. (1986).** Morphogenesis of ductal networks in the mouse prostate. *Biol Reprod* **34,** 961-71.
**Sugimura, Y., Foster, B. A., Hom, Y. K., Rubin, J. S., Finch, P. W., Aaronson, S. A., Hayashi, N., Kawamura, J. and Cunha, G. R.** (1996). Keratinocyte growth factor (KGF) can replace testosterone in the ductal branching morphogenesis of the rat ventral prostate. *Int J Dev Biol* **40**, 941-51.
**Sukegawa, A., Narita, T., Kameda, T., Saitoh, K., Nohno, T., Iba, H., Yasugi, S. and Fukuda, K.** (2000). The concentric structure of the developing gut is regulated by Sonic hedgehog derived from endodermal epithelium. *Development* **127**, 1971-80.
**Thomson, A. A. and Cunha, G. R.** (1999). Prostatic growth and development are regulated by FGF10. *Development* **126**, 3693-701.
**Thomson, A. A., Foster, B. A. and Cunha, G. R.** (1997). Analysis of growth factor and receptor mRNA levels during development of the rat seminal vesicle and prostate. *Development* **124,** 2431-9.
**Thomson, A. A., Timms, B. G., Barton, L., Cunha, G. R. and Grace, O.** C. (2002). The role of smooth muscle in regulating prostatic induction. *Development* **129,** 1905-12.
**Totten, R. S., Heineman, M. W., Hudson, P. B., Sproul, E. E. and Stout, A. P.** (1953). Microscopic differential diagnosis of latent carcinoma of the prostate. *Arch Pathol* **55**, 131-41.
**Wang, Y., Hayward, S. W., Cao, M., Thayer, K. A. and Cunha, G. R.** (2000). Cell differentiation lineage in the prostate. *Differentiation* **68,** 270-9.
**Wojno, K. J. and Epstein, J. I.** (1995). The utility of basal cell-specific anti-cytokeratin antibody (34 beta E12) in the diagnosis of prostate cancer. A review of 228 cases. *Am J Surg Pathol* **19*,*** 251-60.

## Claims

1. Use of a compound which suppresses testosterone or its effect in the manufacture of a medicament for protecting a male patient from possible adverse effects of a treatment involving inhibition of the SHH-signalling pathway in the patient.

2. Use of an inhibitor of the SHH-signalling pathway in the manufacture of a medicament for treating a proliferative disease such as cancer in a patient wherein the patient is administered a compound which suppresses testosterone or its effects in the patient.

3. Use of a compound which suppresses testosterone or its effects in a patient in the manufacture of a medicament for treating a proliferative disease such as cancer in a patient wherein the patient is administered an inhibitor of the SHH-signalling pathway.

4. Use of a combination of an inhibitor of the SHH-signalling pathway and a compound which suppresses testosterone or its effects in a patient in the manufacture of a medicament for treating a proliferative disease such as cancer in a patient.

5. Use according to any one of Claims 1 to 4 wherein the inhibitor of the SHH-signalling pathway is cyclopamine or a derivative thereof.

6. Use according to any one of Claims 1 to 5 wherein the compound which suppresses testosterone or its effects in the patient is any one or more of a GnRH antagonist, a GnRH agonist, an androgen antagonist or a 5α reductase inhibitor.

7. Use according to any one of Claims 1 to 6 wherein the patient is a male, preferably a post-pubescent male.

8. Use according to any one of Claims 1 to 7 wherein the cancer is a cancer in which SHH-signalling plays a role in its growth and/or differentiation.

9. Use according to Claims 2 to 8 wherein the cancer is any of basal cell carcinoma, glioblastoma, medulloblastoma or prostate cancer.

10. Use according to amy of the preceding claims where testosterone is suppressed to castrate levels.

11. A therapeutic system for treating a patient, the system comprising an inhibitor of the SHH-signalling pathway and a compound which suppresses testosterone or its effect in the patient.

12. A composition comprising an inhibitor of the SHH-signalling pathway and a compound which suppresses testosterone or its effect in a patient.

13. A composition according to Claim 12 for use in medicine.

14. A pharmaceutical composition comprising an inhibitor of the SHH-signalling pathway and a compound which suppresses testosterone or its effect in a patient and a pharmaceutically acceptable carrier.

## Patentansprüche

1. Verwendung einer Verbindung, die Testosteron oder seine Effekte unterdrückt, bei der Herstellung eines Medikaments zum Schutz eines männlichen Patienten vor möglichen Nebenwirkungen einer Behandlung, die die Inhibierung des SHH-Signalwegs bei dem Patienten umfasst.

2. Verwendung eines Inhibitors des SHH-Signalwegs bei der Herstellung eines Medikaments für die Behandlung einer proliferativen Erkrankung, wie beispielsweise Krebs, bei einem Patienten, wobei dem Patienten eine Verbindung verabreicht wird, die Testosteron oder seine Effekte bei dem Patienten unterdrückt.

3. Verwendung einer Verbindung, die Testosteron oder seine Effekte bei einem Patienten unterdrückt, bei der Herstellung eines Medikaments für die Behandlung einer proliferativen Erkrankung, wie beispielsweise Krebs, wobei dem Patienten ein Inhibitor des SHH-Signalwegs verabreicht wird

4. Verwendung einer Kombination eines Inhibitors des SHH-Signalwegs und einer Verbindung, die Testosteron oder seine Effekte bei einem Patienten unterdrückt, bei der Herstellung eines Medikaments für die Behandlung einer proliferativen Erkrankung, wie beispielsweise Krebs, bei einem Patienten.

5. Verwendung nach einem der Ansprüche 1 bis 4, wobei der Inhibitor des SHH-Signalwegs Zyklopamin oder ein Derivat davon ist.

6. Verwendung nach einem der Ansprüche 1 bis 5, wobei die Verbindung, die Testosteron oder seine Effekte bei dem Patienten unterdrückt, eine oder mehrere ist von einem GnRH-Antagonisten, einem GnRH-Agonisten, einem Androgenantagonisten oder einem 5 a Reduktaseinhibitor.

7. Verwendung nach einem der Ansprüche 1 bis 6, wobei der Patient ein Mann, vorzugsweise ein postpubertärer Mann ist.

8. Verwendung nach einem der Ansprüche 1 bis 7, wobei der Krebs ein Krebs ist, bei dem SHH-Signalisierung eine Rolle bei seinem Wachstum und/oder seiner Differenzierung spielt.

9. Verwendung nach Anspruch 2 bis 8, wobei der Krebs einer ist von Basalzellenkrebs, Glioblastom, Medulloblastom oder Prostatakrebs.

10. Verwendung nach einem der vorangehenden Ansprüche, wobei Testosteron auf Kastratenniveaus unterdrückt wird.

11. Therapeutisches System für die Behandlung eines Patienten, wobei das System einen Inhibitor des SHH-Signalwegs und eine Verbindung aufweist, die Testosteron oder seine Effekte bei dem Patienten unterdrückt.

12. Zusammensetzung, die einen Inhibitor des SHH-Signalwegs und eine Verbindung aufweist, die Testostron oder seine Effekte bei einem Patienten unterdrückt.

13. Zusammensetzung nach Anspruch 12 zur Verwendung in der Medizin.

14. Pharmazeutische Zusammensetzung, die einen Inhibitor des SHH-Signalwegs und eine Verbindung, die Testosteron oder seine Effekte bei einem Patienten unterdrückt, und einen pharmazeutisch akzeptablen Träger aufweist.

## Revendications

1. Utilisation d'un composé qui élimine la testostérone ou ses effets dans la fabrication d'un médicament destiné à protéger un patient de sexe masculin des possibles effets indésirables d'un traitement impliquant l'inhibition de la voie de signalisation SHH chez le patient.

2. Utilisation d'un inhibiteur de la voie de signalisation SHH dans la fabrication d'un médicament destiné à traiter une maladie proliférative telle que le cancer chez un patient, dans laquelle on administre au patient un composé qui élimine la testostérone ou ses effets chez le patient.

3. Utilisation d'un composé qui élimine la testostérone ou ses effets chez un patient dans la fabrication d'un médicament destiné à traiter une maladie proliférative telle que le cancer chez un patient, dans laquelle on administre au patient un inhibiteur de la voie de signalisation SHH.

4. Utilisation d'une combinaison d'un inhibiteur de la voie de signalisation SHH et d'un composé qui élimine la testostérone ou ses effets chez un patient dans la fabrication d'un médicament destiné à traiter une maladie proliférative telle que le cancer chez un patient.

5. Utilisation selon l'une quelconque des revendications 1 à 4, dans laquelle l'inhibiteur de la voie de signalisation SHH est la cyclopamine ou un dérivé de celle-ci.

6. Utilisation selon l'une quelconque des revendications 1 à 5, dans laquelle le composé qui élimine la testostérone ou ses effets chez le patient est l'un quelconque ou plusieurs parmi un antagoniste de la GnRH, un agoniste de la GnRH, un antagoniste des androgènes ou un inhibiteur de la 5α-réductase.

7. Utilisation selon l'une quelconque des revendications 1 à 6, dans laquelle le patient est de sexe masculin, de préférence un homme post-pubère.

8. Utilisation selon l'une quelconque des revendications 1 à 7, dans laquelle le cancer est un cancer dans lequel la voie de signalisation SHH joue un rôle dans sa croissance et/ou sa différentiation.

9. Utilisation selon les revendications 2 à 8, dans laquelle le cancer est l'un parmi le carcinome basocellulaire, le glioblastome, le médulloblastome ou le cancer de la prostate.

10. Utilisation selon l'une quelconque des revendications précédentes, où la testostérone est supprimée jusqu'à des niveaux de castration.

11. Système thérapeutique destiné à traiter un patient, le système comprenant un inhibiteur de la voie de signalisation SHH et un composé qui élimine la testostérone ou ses effets chez le patient.

12. Composition comprenant un inhibiteur de la voie de signalisation SHH et un composé qui élimine la testostérone ou ses effets chez un patient.

13. Composition selon la revendication 12 destinée à un usage en médecine.

14. Composition pharmaceutique comprenant un inhibiteur de la voie de signalisation SHH et un composé qui élimine la testostérone ou ses effets chez un patient et un excipient pharmaceutiquement acceptable.
